Europäisches Patentamt
European Patent Office
Office européen des brevets

(19) (11) Publication number: **0 010 366**
**B1**

(12) # EUROPEAN PATENT SPECIFICATION

(45) Date of publication of patent specification: **16.02.83**

(21) Application number: **79301990.2**

(22) Date of filing: **25.09.79**

(51) Int. Cl.$^3$: **C 07 F 9/09,** C 07 F 9/40, C 08 G 79/14, B 01 J 39/12, B 01 J 45/00, B 01 J 31/08, C 08 K 5/49

(54) **Solid organometallic inorganic polymers and their application.**

(30) Priority: **26.09.78 US 945971**
**17.10.78 US 952228**
**04.12.78 US 966197**
**29.01.79 US 7275**
**30.05.79 US 43810**
**02.07.79 US 54097**
**02.07.79 US 54107**
**24.07.79 US 60076**
**24.07.79 US 60077**
**24.07.79 US 60078**
**24.07.79 US 60079**
**24.07.79 US 60249**
**24.07.79 US 60250**

(43) Date of publication of application:
**30.04.80 Bulletin 80/9**

(45) Publication of the grant of the patent:
**16.02.83 Bulletin 83/7**

(84) Designated Contracting States:
**AT BE CH DE FR GB IT NL SE**

(56) References cited:
**DE - A - 2 614 356**
**GB - A - 539 293**
**GB - A - 1 406 419**

(73) Proprietor: **OCCIDENTAL RESEARCH CORPORATION**
**2100 S.E. Main Street**
**Irvine, California 92714 (US)**

(72) Inventor: **DiGiacomo, Peter Michael**
**27671 Estepona**
**Mission Viego California 92691 (US)**
Inventor: **Dines, Martin Benjamin**
**19162 Barrett Lane**
**Santa Ana California 92705 (US)**
Inventor: **Parziale, Victor Ernest**
**5013 Verano Place**
**Irvine California 92715 (US)**

(74) Representative: **Jack, Bruce James et al,**
**FORRESTER & BOEHMERT Widenmayerstrasse 4/I**
**D-8000 Munchen 22 (DE)**

(56) References cited:
**US - A - 3 231 347**
**US - A - 3 615 807**
**US - A - 3 634 479**

**Journal of Inorganic Nuclear Chemistry. Volume 40, pages 1113 to 1117 (1978)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 010 366 B1

Courier Press, Leamington Spa, England

# Solid organometallic inorganic polymers and their application

*Background of the Invention*

The present invention is directed to solid inorganic polymers having organo groups anchored to the surfaces of the polymers. The majority of the polymers formed are layered crystals which display intercalation activity.

The interface surfaces of solids, whether amorphous, crystalline, or semicrystalline, are responsive regions of chemical and physical action. In many practical chemical and physical phenomena, such as absorption, corrosion inhibition, heterogeneous catalysis, lubrication, ion exchange activity, adhesion and wetting and electrochemistry, activity occurs as a consequence of the presence of a definable solid surface.

Many inorganic solids crystallize with a layered structure and some could present sites for anchoring active groups. In this form, sheets or slabs with a thickness of from one to more than seven atomic diameters lie upon one another. With reference to FIG. 1, strong ionic or covalent bonds characterize the intrasheet structure, while relatively weak van der Waals or hydrogen bonding occurs between the interlamellar basal surfaces in the direction perpendicular to their planes. Some of the better known examples are prototypal graphite, most clay minerals, and many metal halides and sulfides. A useful characteristic of such materials is the tendency to incorporate "guest" species in between the lamella.

In this process, designated "intercalation" the incoming guest molecules, as illustrated in FIG. 2, cleave the layers apart and occupy the region between them. The layers are left virtually intact since the crystals simply swell in one dimension, i.e., perpendicular to the layers. If the tendency to intercalate is great, then the host-layered crystal can be thought of as possessing an internal "super surface" in addition to its apparent surface. In fact, this potential surface will be greater than the actual surface by a factor of the number of lamella composing the crystal. This value is typically on the order of $10^2$ to $10^4$. Although edge surface is practically insignificant compared to basal surface, it is critical in the rate of intercalation, since the inclusion process always occurs via the edges. This is because bonding within the sheet is strong and, therefore, basal penetration of the sheets is an unlikely route into the crystal.

In graphite, the function of the host is essentially passive. That is on intercalation, the host serves as the matrix or surface with which the incoming guest molecules interact, but throughout the process and on deintercalation the guests undergo only minor perturbation.

In order for a more active process to occur during intercalation, such as selective complexation of catalytic conversion, specific groups must be present which effect such activity.

An approach in which catalytically active agents have been intercalated into graphite or clays for subsequent conversions has been described in "Advanced Materials in Catalysis, "Boersma, Academic Press, N.Y. (1977), Burton et al, editors, and "Catalysis in Organic Chemistry," Pinnavia, Academic Press, N.Y. (1977), G.V. Smith, editor.

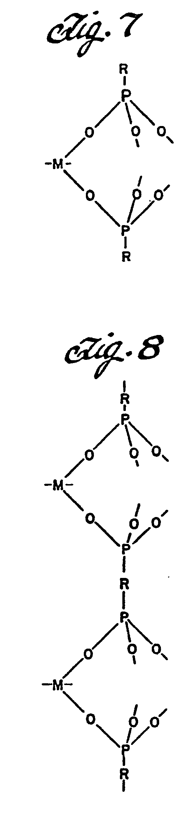

One of the few layered compounds which have potential available sites is zirconium phosphate $Zr(O_3POH)_2$. It exists in both amorphous and crystalline forms which are known to be layered. In the layered structure, the site-site placement on the internal surfaces is about 5.3Å, which leads to an estimated 25Å$^2$ area per site. This area can accommodate most of the functional groups desired to be attached to each site. The accepted structure, symbolized projection of a portion of a layer of this inorganic polymer and a representation of an edge view of two layers, are shown respectively in FIGS. 3, 4 and 5.

Besides the advantageous structural features of zircoinium phosphate, the material is chemically and thermally stable, and nontoxic.

Quite a bit of work has been conducted on the zirconium phosphate, mainly because it has been found to be a promising inorganic cation exchanger for alkali, ammonium and actinide ions, Alberti, "Accounts of Chemistry Res." *11* 163, 1978. In addition, some limited work has been described on the reversible intercalation behavior of layered zirconium phosphate toward alcohols, acetone, dimethylformamide and amines, Yamaka and Koisuma, "Clay and Clay Minerals" 23, 477 (1975) and Michel and Weiss, "Z. Natur," *20*, 1307 (1965). S Yamaka described the reaction of this solid with ethylene oxide, which does not simply incorporate between the layers as do the other organics, but rather was found to irreversibly react with the acid hydroxyls to form a covalent bonded product, Yamaka, "Inorg. Chem." *15*, 2811, (1976). This product is composed of a bilayer of anchored ethanolic groups aimed into interlayers. The initial layer-layer repeat distance is expanded from about 7.5Å to 15Å, consistent with the double layer of organics present. The overall consequence of this reaction is to convert inorganic acid hydroxyls to bound organic alkanol groups.

A very recently reported effort in the field is Alberti, et al., "J. Inorg. Nucl. Chem.," *40*, 1113 (1978). A method similar to that of this invention for the preparation of zirconium bis(benzenephosphonate), zirconium bis(hydroxymethanephosphonate) monohydrate, and zirconium bis(monoethylphosphate) is described, with descriptions of the properties for these products.

Following the Alberti publication, a paper by Maya appeared in "Inorg. Nucl. Chem. Letters," *15*, 207 (1979), describing the preparation, properties and utility as solid phases in reversed phase liquid

chromatography for the compounds $Zr(O_3POC_4H_9)_2 \cdot H_2O$, $Zr(O_3POC_{12}H_{25})_2$ and $Zr(O_3POC_{14}H_{21})_2$.

All of the compositions described herein can be useful in gas phase, liquid phase, gas liquid, reversed phase, and bulk and thin layer chromatography. The compounds can also be useful as hosts and carriers for organic molecules and especially biologically active organic molecules. They are also useful as catalysts or as supports for catalysts. For example, they can be used in an analogous fashion to the compositions which are discussed by Bailar, "Heterogenizing Homogeneous Catalysts", *Catalysis Reviews—Sci. & Eng.*, 10(1) 17—35 (1974) and Hartley and Vezey, "Supported Transition Metal Complexes as Catalysts", *Advances in Organometallic Chemistry*, 15, 189—235 (1977).

The present invention provides solid inorganic polymers that are characterised by having basic structural units conforming to the formula:

$$M(O_3PO_xR)_n$$

in which M is one or more of the tetravalent elements tungsten, uranium, titanium, thorium, tellurium, tin, silicon, rubidium, plutonium, praseodymium, lead, osmium, niobium, molybdenum, manganese, iridium, hafnium, germanium and cerium, and especially is one or more of titanium, molybdenum, tin, cerium, hafnium, lead, thorium and uranium; R is one or more organo, acyclic, alicyclic, heteroacyclic, heterocyclic or aromatic groups, x is 0 or 1; and n is 1 or 2, provided that when n is 1, R is terminated with an unsubstituted phosphate or phosphonate group and contains at least two carbon atoms separating the phosphorus atoms.

The inorganic polymers of this invention have organo groups covalently bonded to phosphorus. The phosphorus atoms are, in turn, covalently bonded by an oxygen linkage to tetravalent element atoms. When formed in a layered crystalline state, they provide the organo groups on all of the apparent and interlamellar surfaces.

The polymers may be prepared by a liquid media reaction in which the ion of at least of one of said tetravalent elements is reacted with at least one organophosphorus acid compound of the formula:

$$((HO)_2OP)_mR$$

wherein m is 1 or 2 and R is an organo group covalently coupled to the phosphorus atom, and wherein when m is 2, R contains at least two carbon atoms and is directly or indirectly coupled to the phosphorus atoms through different carbon atoms whereby the two phosphorus atoms are separated by at least two carbon atoms. The molar ratio of phosphorus to the tetravalent element is 2 to 1. Reaction preferably occurs in the presence of an excess of the organic phosphorus acid compound and the ion is provided as a compound soluble in the liquid media, for instance as a soluble salt $MX_4$, where X is an anion such as halide, $HSO_4^{-1}$, $SO_4^{-2}$, $O_2C—CH_3^{-1}$, $NO_3^{-1}$, or $O^{-2}$.

Where only one species of an organophosphorus acid compound is provided as the reactant with the tetravalent element compound, the end product will have the empirical formula $(M(O_3PO_xR)_n$. Phosphoric and/or phosphorous acid can also be present as reactive diluents to form part of the solid inorganic polymeric structure which is the product of the reaction.

The products formed are layered crystalline to amorphous in nature. For all products, the R groups can be directly useful or serve as intermediates for the addition or substitution of other functional groups. When the product is crystalline and n is 1, that is, when the product derives from an organophosphorus acid compound in which m is 2 in the foregoing formula, cross-linking between the interlamellar layers occurs.

The normal liquid medium is water. However, organic solvents, particularly ethanol, can be employed where water will interfere with the desired reaction. Preferably, the solvent is the solvent in which the organophosphorus acid compound is prepared. Where the organophosphorus acid compound has a sufficiently low melting point, it can serve as the liquid medium.

The metathesis reaction occurs at temperatures up to the boiling point of the liquid media at the pressures involved, typically from ambient to about 150°C and more preferably from ambient to about 100°C. While formation of the solid inorganic polymer is almost instantaneous, the degree of crystallinity of the product can be increased by refluxing the reaction products for times from about 5 to 15 hours. Crystallinity is also improved by employing a sequestering agent for the tetravalent ion.

*Brief Description of the Drawings*

FIG. 1 illustrates a layered microcrystal. Each lamellar slab is formed of strong covalent bonds and has a thickness of about 10 atoms.

FIG. 2 illustrates intecalation where the interlayer distance is shown as "d."

FIG. 3 illustrates the accepted structure for zirconium phosphate and spacing between layers. The dashed lines between zirconium (Zr) atoms is to establish the plane between them. In the drawing, P is phosphorous, O is oxygen and water of hydration is shown.

FIG. 4 illustrates a projection of the zirconium plane showing accepted spacing between Zr atoms and the available linkage area.

FIG. 5 is a symbolized depiction of spaced zirconium phosphate layers showing covalently bonded hydroxyl groups and water of hydration.

FIG. 6 illustrates an exchange reaction between anchored groups "A" and groups "B" to be substantially for groups "A", and —∨ represents the portion of the organo group linking the terminal group "A" or "B" to the crystals or the organophosphorus acid compound reactant.

FIG. 7 shows the basic structural unit of the inorganic polymer wherein n is 2 and wherein P is phosphorus, O is oxygen, M is tetravalent element and R is the organo group.

FIG. 8 shows the basic structural unit of the inorganic polymer wherein n is 1 and wherein P is a phosphorus atom, O is an oxygen atom, M is a tetravalent element and R is the organo group.

*Detailed Description*

The present invention provides solid inorganic polymers that may exist in layered crystalline to amorphous state and that may be formed by the liquid phase metathesis reaction of at least one tetravalent ion with at least one organophosphorus acid compound.

Tetravalent ions useful herein are analogous to $Zr^{+4}$ in the process to make zirconium phosphate and phosphonate analogs and are elements with approximately the same ionic radius as $Zr^{+4}$ (0.8Å); for example, the following:

| | | | | | | | |
|---|---|---|---|---|---|---|---|
| $Zr^{+4}$ | 0.80Å | $Te^{+4}$ | 0.81 | $Pr^{+4}$ | 0.94 | $Mn^{+4}$ | 0.5 |
| $W^{+4}$ | 0.66 | $Sn^{+4}$ | 0.71 | $Pb^{+4}$ | 0.92 | $Ir^{+4}$ | 0.66 |
| $U^{+4}$ | 0.89 | $Si^{+4}$ | 0.41 | $Os^{+4}$ | 0.67 | $Hf^{+4}$ | 0.81 |
| $Ti^{+4}$ | 0.68 | $Ru^{+4}$ | 0.65 | $Nb^{+4}$ | 0.67 | $Ge^{+4}$ | 0.53 |
| $Th^{+4}$ | 0.95 | $Pu^{+4}$ | 0.86 | $Mo^{+4}$ | 0.68 | $Ce^{+4}$ | 1.01 |

The majority of the polymeric reaction products formed from the above elements are found to be layered crystalline or semicrystalline in nature and, as such, provide layered structures similar to zirconium phosphates. The remainder are amorphous polymers possessing a large quantity of available pendant groups similar to silica gel.

By the term "organophosphorus acid compound," as used herein, there is meant a compound of the formula:

$$((HO)_2OP)_mR$$

wherein m is 1 or 2, R is any group which will replace a hydroxyl of phosphoric acid and/or the hydrogen or phosphorus acid and couple to the acid by a covalent bond. Coupling to the acid may be through carbon, oxygen, silicon, sulfur, nitrogen and the like. Coupling through carbon or an oxygen-carbon group is presently preferred.

When, in the organophosphorus compound, m is 2, the end product occurs in the bis configuration. In this configuration, R must contain two or more carbon atoms, preferably from two to about 20 carbon atoms, such that at least two carbon atoms separate the phosphorus atoms. In such a configuration R can be considered to have a phosphate or phosphonate termination. In this bis configuration, no single carbon atom is bound directly or indirectly to more than one $(PO(OH)_2)$ group. Thus, the groups which link to the tetravalent element have the basic structural formula:

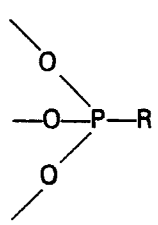 or 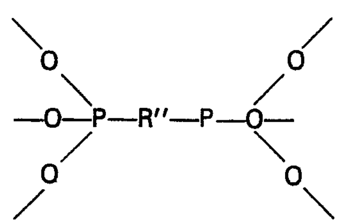

wherein R'' is a bis group containing at least two carbon atoms bonded directly or indirectly to phosphorus and such that no phosphorus atoms are bonded directly or indirectly to the same carbon atom. The basic structures of the inorganic polymer forms are shown in FIGS. 7 and 8.

When coupling is through carbon, the organophosphorus acid compound is an organophosphonic acid and the product a phosphonate. When coupling is through oxygen-carbon, the organophosphorus acid compound is an organophosphoric monoester acid and the product a phosphate.

The general reaction for phosphonic acids alone is shown in equation (1) below and for monoesters of phosphoric alone by equation (2).

$$M^{+4} + 2(HO)_2OPR \rightarrow M(O_3P{-}R)_2 + 4H^+ \quad (1)$$

$$M^{+4} + 2(HO)_2OP{-}OR' \rightarrow M(O_3P{-}OR')_2 + 4H^+ \quad (2)$$

wherein R' is the remainder of the organo group.

The product contains phosphorus to tetravalent element in a molar ratio of about 2 to 1, and the empirical formula for the product would show all groups bound to phosphorus.

As used herein, R is an acyclic group, heteroacyclic group, alicyclic group, aromatic group or heterocyclic group.

The term "acyclic group," as used herein, means a substituted or unsubstituted acyclic group. The term "acyclic" includes saturated and unsaturated aliphatics which can be straight chain or branched chain. The "aliphatics" include alkyl, alkenyl and alkynyl.

The term "heteroacyclic group," as used herein, means an acyclic group containing one or more heteroatoms in the chain selected from oxygen, nitrogen and sulfur. The heteroatoms can be the same or different in each chain and usually the number of heteroatoms is one, two or three.

The term "alicyclic group," as used herein, means a substituted or unsubstituted alicyclic group. The term "alicyclic" includes saturated and unsaturated cyclic aliphatics.

The term "aromatic groups," as used herein, means a substituted or unsubstituted aromatic group. The term "aromatic" includes phenyl, naphthyl, biphenyl, anthracyl and phenanthryl.

The term "heterocyclic group," as used herein, means a substituted or unsubstituted heterocyclic group. The term "heterocyclic" means an alicyclic or aromatic group containing one or more hetero atoms in the ring selected from oxygen, nitrogen and sulfur. The heteroatom can be the same or different in each ring and usually the number of heteroatoms is one, two or three.

The terms "substituted acyclic," "substituted heteroacyclic," "substituted alicyclic," "substituted aromatic" and "substituted heterocyclic," as used herein, mean an acyclic, heteroacyclic, alicyclic, aromatic or heterocyclic group substituted with one or more of the groups selected from alkyl, alkenyl, alkynyl, alkoxy, alkenyloxy, alkynyloxy, alkylthio, alkenylthio, alkynylthio, halo, oxo, hydroxy, carbonyl, carboxy, alkylcarbonyloxy, alkylcarbonyl, carboxyalkyl, thio, mercapto, sulfinyl, sulfonyl, inino, amino, cyano, nitro, hydroxyamino, nitroso, cycloalkyl, cycloalkaryl, aryl, aralkyl, alkaryl, aryloxy, arylalkoxy,, alkaryloxy, arylthio, aralkylthio, alkarylthio, arylamino, aralkylamino and alkarylamino.

In general, the organo group should occupy no more than about 24Å$^2$ for proper spacing. This limitation is imposed by the basic crystal structure of zirconium phosphate. Referring to FIG. 4, a spacing of 5.3Å is shown between zirconium atoms in the zirconium plane of a crystal a total area of about 24Å$^2$ is shown for the space bounded by zirconium atoms. It follows that any group anchored on each available site cannot have an area much larger than the site area and maintain the layered structure.

This limitation can be avoided through the use of a combination of larger and smaller groups, i.e., mixed components. If some of the sites are occupied by groups which have an area much less than about 24Å$^2$, adjacent groups can be somewhat larger than 24Å$^2$ and still maintain the layered structure of the compound.

The cross-sectional area which will be occupied by a given organo group can be estimated in advance of actual compound preparation by ue of CPK space filling molecular models (Ealing Company) as follows: A model of the alkyl or aryl chain and terminal group is constructed, and it is situated on a scaled pattern of a hexagonal array with 5.3Å site distances. The area of the group is the projection area on this plane. Some areas which have been determined by this procedure are listed in Table I.

TABLE I

| Moiety | Minimum Area (Å$^2$) | Moiety | Minimum Area (Å$^2$) |
|---|---|---|---|
| Alkyl chain | 15 | Isopropyl | 22.5 |
| Phenyl | 18 | t-butyl | 25 |
| Carboxyl | 15 | Chloromethyl | 14 |
| Sulfonate | 24 | Bromoethyl | 17 |
| Nitrile | 9 | Diphenylphosphine | 50 (approx.) |
| Morpholinomethyl | 21 | mercaptoethyl | 13.5 |
| Trimethylamino | 25 | | |

The process for the formation of the novel inorganic polymers is a metathesis reaction conducted in the presence of a liquid medium receptive to the tetravalent ion at a temperature up to the boiling point of the liquid medium, preferably from ambient to about 150°C and, more preferably, to about 100°C at the pressure employed.

While water is the preferred liquid medium with phosphorus, as most of the organophosphorus acid compounds are hygroscopic, an organic solvent, such as ethanol can be employed, where water interferes with the reaction. There need only be provided a solvent for the organophosphorus acid compound since the tetravalent ion can be dispersed as a solid in the solvent for slow release of the ion for reaction with the organophosphorus acid compound. If it has a sufficiently low melting point, the organophosphorus acid compound can serve as a solvent. Typically, the liquid medium is the liquid medium in which the organophosphorus acid is formed.

For complete consumption of the tetravalent compound, the amount of acid employed should be sufficient to provide two moles of phosphorus per mole of tetravalent element. An excess is preferred. Phosphorous acid and/or phosphoric acid, if present, enters into the reaction and provides an inorganic polymer diluted in respect of the organo group in proportion to the amount of phosphorous or phosphoric acid employed.

Reaction is virtually instantaneous at all temperatures leading to precipitation of layered crystalline, semicrystalline or amorphous solid inorganic polymer.

The amorphous phase appears as a gel similar to silica gel. The gel can be crystallized by extended reflux in the reaction medium, usually from about 5 to about 15 hours. The semicrystalline product is characterized by a rather broad X-ray powder pattern.

The presence of sequestering agents for the tetravalent ion slows down the reaction and also leads to more highly crystalline products. For instance, an enhancement of crystallinity was obtained in the reaction of thorium nitrate with 2-carboxyethyl phosphonic acid. Nitrate ion is a sequestering agent for thorium and the rate of formation of this product is slow and the product polymer quite crystalline. The presence of the nitrate ion results in slow release of the tetravalent ion for reaction with the phosphonic acid, resulting in an increase in crystallinity.

As compared to zirconium phosphate forming crystals of 1—5 microns, crystals of 100 to greater than 1000 microns in size have been prepared in accordance with the invention.

A critical property for many of the likely uses of the products is their thermal stability. This is because deficiencies in activity can be compensated for by reasonable increases in operating temperature. A standard method for thermal characterization is thermal gravimetric/differential thermal analysis (TGA/DTA). These techniques indicate changes in weight and heat flow of substances as a function of temperature. Thus, decomposition and phase changes can be monitored as temperature increases.

Zirconium phosphate itself is quite a stable material. Interlayer water is lost at about 100°C, and a second dehydration involving the phosphates occurs above 400°C. The practical ion-exchanging abilities are lost in this step.

The inorganic phosphorus-containing polymers of this invention are also stabilized toward thermal decomposition as compared to pure inorganic analogs as a result of the fixation and separating effect of the inorganic support.

While not bound by theory, phosphates probably decompose like carboxylic esters to yield acid and unsaturates, whereas phosphonates likely form radicals by homolytic cleavage.

Besides providing the suitability of such compounds in elevated temperature applications, the TGA analysis affirms that covalent bonding occurs to phosphorus. This is because normal intercalative interactions are reversed within 10° to 100°C above the boiling point of the guest.

The process of this invention permits a wide variety of inorganic polymers to be formed having the characteristic of the organo group protected by the inorganic polymer structure and with subsequent exchange or substitution reactions, the formation of other inorganic polymers. Polymers formed may be block, random and the like.

For instance, a mixture of phenyl phosphonic acid and phosphorous acid can be simultaneously reacted with a tetravalent ion to yield a single solid phase. The interlamellar distance exhibited is the same as the tetravalent element phenyl phosphonate, rather than the normal spacing for the tetravalent element phosphite. This establishes that the largest group should determine interlamellar distance and indicated that a discrete tetravalent element phosphate phase was not present. Evidence of a change in chemical environment of P—H bond is established by infrared analysis. In infrared analysis of a tetravalent metal phosphite, P—H stretching is observed as a sharp band and in the mixed solid compound, the band is shifted slightly and broadened.

Another route is to exchange one pendant group for another. While not bound by theory, the present expected points of exchange are at the periphery of the crystal and are schematically illustrated in FIG. 6. Such bifunctional materials exhibit the quality of providing terminal groups for attracting species for intercalation and then interaction with the internal groups.

The reaction of bis acids with tetravalent ions permits interlamellar cross-linking by a reaction such as

$$(HO)_2OPCH_2CH_2OP(OH)_2 + M^{+4} \rightarrow$$ 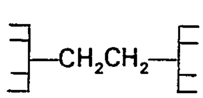

where, as in FIG. 6, 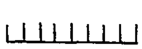 represents the interlamellar layers to which the alkyl group is anchored.

As with all organo groups, for the bis configuration at least two carbona toms are present, preferably from two to twenty atoms, and the phosphorus atoms are linked directly or indirectly to different carbon atoms. Since size of the linking group will control and fix interlamellar spacing, there is provided effective laminar sieves of fixed spacing for application analogous to that of molecular sieves.

Ion exchange activity of the compounds herein can be established with compounds having pendant carboxylic acid groups. Prepared thorium 2-carboxyethyl phosphonate was established to have an interlayer distance of 14.2Å. When intercalated to form its n-hexylammonium salt, the interlayer distance increased. When sodium is taken up layer spacing increases. X-ray and infrared data indicate the highly crystalline inorganic polymer to behave as expected for a carboxylic acid, with behavior analogous to ion exchange resins, except that both external and internal surfaces are functional, establishing them as super surface ion exchange resins. Moreover, since the inorganic polymers can be prepared as microcrystalline powders, diffusion distances are short.

As summarized in Table II, nitrile and mercapto anchored groups show the ability to take up silver and copper ions at room temperature for catalytic activity.

TABLE II

| Anchored Group | Metal Ion | Loading MMole Metal MMole $M^{+4}$ |
|---|---|---|
| –0 ~ CN | 0.1 M Ag+ | 0.20 |
| ~ SH | 0.1 M Ag+ | 1.0 |
| –0 ~ CN | 0.1 M Cu++ | 0.10 |
| –0 ~ CN | 0.1 M Cu++ | 0.10 |
| | 0.5 M HOAc | |
| | 0.5 M NaAc | |

~ = groups formed of carbon and hydrogen.

Ac = acetate radical

The alternative to catalytic utility is to attach the metals to the organophosphorus acid prior to reaction with the soluble tetravalent compound.

The high surface area of the crystalline products also makes them useful for sorption of impurities from aqueous and nonaqueous media.

Another utility is as an additive to polymeric compositions. Simialr to the high aspect ratio provided by solids such as mica which improve the stress strain properties of the polymers, the powdered inorganic polymer products of the invention can serve the same function and add features. By the presence of reactive end groups on the bonded organo groups, chemical grafting to the polymer network can be achieved to increase composite crystallinity and elevating heat distortion temperature. In addition, the presence of phosphorus induces flame retardant properties, as would bound halogen.

Still other utilities include solid lubricants which behave like mica, graphite and molybdenum disulfide; solid slow release agents where intercalated materials can be slowly leached or released from the internal layers of the crystals; substance displaying electrical, optical phase or field changes with or without doping, and the like.

While nowise limiting, the following examples are illustrative of the preparation of solid inorganic polymers of this invention and some of their utilities.

In the examples conducted in the atmosphere, no extraordinary precautions were taken concerning oxygen or moisture. Reagents were usually used as received from suppliers. The products formed are insoluble in normal solvents and do not sublime. However, the combined weight of yield data, spectroscopy, elemental analyses, TGA and power diffraction results confirm the compositions reported with good reliability.

X-ray powder patterns were run on a Phillips diffractometer using CuK radiation.

Thermal analyses were conducted on a Mettler Instrument. Infrared spectra were obtained with a Beckman Acculab spectrophotometer.

Surface areas were determined using both dynamic flow method, on a Quantasorb Instrument, and also with a vacuum static system on a Micromeritic device. Both employ a standard BET interpretation of nitrogen coverage.

Titrations were carried out in aqueous or alcoholic medium. A standard combination electrode and an Orion Ionalyzer pH meter were used for pH determination. The titration of the solid interlamellar anchored materials is analogous to the titration of an ion exchange resin.

Example 1

Preparation of: $Th(O_3PCH_2Cl)_2$

To a reaction flask was added 10.22 g of an aqueous solution of about 85 percent by weight $H_2PO_3CH_2Cl$ and an aqueous solution containing 6.619 g of $Th(NO_3)_4 \cdot 4H_2O$.

Upon mixing the solutions, a white precipitate formed almost immediately. The reaction mixture was then refluxed for about a day to enhance the layered structure of the crystals formed.

Following refluxing, the solid precipitate was separated from the liquid by filtration. The recovered solid was washed with successive washes of water, acetone and ether. The resulting solid was allowed to dry in an oven at about 55°C.

Upon infrared analysis, the solid was shown to be $Th(O_3PCH_2Cl)_2$.

Elemental analysis of the recovered product provided the following results: 4.62 C; 1.46% H; and 12.65% Cl. An X-ray powder diffraction pattern showed the compound to be crystalline having an interlayer spacing of 10.5Å.

Example 2

Preparation of:

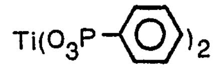

In a suitable reaction vessel containing 100 ml of water was added 5.665 g of

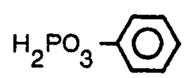

and 8.087 g of a 30 percent by weight aqueous solution of $TiOCl_2$ and 1 ml of 38 percent hydrochloric acid.

Upon mixing the reagents a precipitate appeared almost immediately upon mixing. The mixture containing the reactants was refluxed overnight under reflux conditions. Following refluxing, the resultant mixture was filtered and the precipitate separated washed with water. The precipitate was then dried in an oven at about 50°C for a few hours until a constant weight was obtained. The final weight of the precipitate,

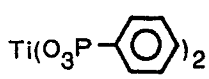

was 5.722 g. The theoretical yield was 6.480 g which provided an 88.3 percent yield for the reaction.

An X-ray powder diffraction pattern showed the compound to be crystalline having an interlayer spacing of 15.2Å.

Examples 3—27

Using the method outlined in Example 1 or Example 2, the inorganic polymers listed in the following Table III were prepared. In Table III the compound produced, phosphorus-containing reagent, $M^{+4}$ salt, elemental analysis of product, interlayer spacing and product structure are listed. The weights are listed in grams. When the $M^{+4}$ salt is listed as $Th(NO_3)_4$ it is meant $Th(NO_3)_4 \cdot 4H_2O$ was used in the example. In the column designated "other" the element analyzed is listed following its determined weight percentage. The interlayer spacings for the compounds were determined using X-ray powder diffraction techniques. In the column designated structure the letters C, S and A are used to designate crystalline, semicrystalline and amorphous configurations respectively.

TABLE III

| Ex. | Compound Produced | Product Wt. g | Phosphorus Reagent Used | Acid Wt. g | $M^{+4}$ Salt Wt. g | Weight Percent | | | | I.S. A | Str. |
|---|---|---|---|---|---|---|---|---|---|---|---|
| | | | | | | C | H | Other | $M^{+4}$ | | |
| 3 | $U(O_3POCH_2CH_2CN)_2$ | 5.02 | $BaO_3PO$ $-CH_2CH_2CN$ | 5.8 | $UCl_4$ 3.8 | 11.67 | 2.12 | | | 13.8 | S–C |
| 4 | $Th(O_3PCH_2Cl)_2$ | 6.0 | $H_2O_3PCH_2Cl$ | 10.0 | $Th(NO_3)_4$ 6.6 | 4.62 | 1.46 | 12.65 Cl | | 10.5 | C |
| 5 | $Pb(O_3PCH_2Cl)_2$ | 5.1 | $H_2O_3PCH_2Cl$ | 12.2 | $PbO_2$ 3.9 | 3.65 | 7.2 | | | 9.83 | C |
| 6 | $U(O_3PCH_2Cl)_2$ | 0.4 | $H_2O_3PCH_2Cl$ | 0.55 | $UCl_4$ 0.34 | | | | | 10.0 | C |
| 7 | $Ti(O_3PCH_2Cl)_2$ | 5.9 | $H_2O_3PCH_2Cl$ | 4.74 | $TiCl_4$ 4.4 | 6.37 | 2.79 | 16.36 Cl | | 10.4 | C |
| 8 | $Th(O_3PC_6H_5)_2$ | 3.44 | $H_2O_3PC_6H_5$ | 2.03 | $Th(NO_3)_4$ 3.48 | | | | | 14.7 | C |
| 9 | $Ce(O_3PC_6H_5)_2$ | 1.3 | $H_2O_3PC_6H_5$ | 0.9 | $Ce(HSO_4)_2$ 0.7 | | | | | 15.5 | C |
| 10 | $Ti(O_3PC_6H_5)_2$ | 1.94 | $H_2O_3PC_6H_5$ | 1.67 | $TiCl_4$ 1.0 | | | | | 15.2 | C |
| 11 | $Th(O_3PCH_2CH_2COOH)_2$ | 1.97 | $H_2O_3PCH_2$ $-CH_2COOH$ | 1.9 | $Th(NO_3)_4$ 2.3 | | | | | 14.2 | C |
| 12 | $Th(p\text{-}O_3POC_6H_4NO_2)_2$ | 1.66 | $H_2O_3POC_6H_4$ $-NO_2$ | 3.03 | $Th(NO_3)$ 2.21 | | | | | 16.4 | C |
| 13 | $Ti_{1/2}Th_{1/2}(O_3PC_6H_5)_2$ | 2.35 | $H_2O_3PC_6H_5$ | 1.17 | $TiOCl_2$ 1.66 $Th(NO_3)_4$ 2.05 | 22.14 | 2.34 | 9.64 P | 5.50 Ti 22.1 Th | 16.0 | C |

TABLE III (Continued)

| Ex. | Compound Produced | Product Wt. g | Phosphorus Reagent Used | Acid Wt. g | $M^{+4}$ Salt Wt. g | Weight Percent | | | | I.S. A | Str. |
|---|---|---|---|---|---|---|---|---|---|---|---|
| | | | | | | C | H | Other | $M^{+4}$ | | |
| 14 | $Th(O_3PCH_3)_2$ | 1.7 | $H_2O_3PCH_3$ | 0.845 | $Th(NO_3)_4$ 2.25 | | | | | | |
| 15 | $Th(O_3PCH_2OH)_2$ | 1.13 | $H_2O_3PCH_2OH$ | 0.552 | $Th(NO_3)_4$ 1.35 | 2.30 | 2.26 | | | 9.51 | C |
| 16 | $Th(O_3PC_{18}H_{37})_2$ | 1.1 | $(CH_3O)_2OP$ $-C_{18}H_{37}$ | 0.952 | $Th(NO_3)_4$ 0.726 | 46.57 | 8.43 | | 20.9 | 42 | C |
| 17 | $Ti(p\text{-}O_3PC_6H_4-OCH_3)_2$ | 0.9 | $H_2O_3PC_6H_4$ $-OCH_3$ | 0.979 | $TiOCl_2$ 1.168 | 40.12 | 3.55 | 14.6 P | 11.2 | 18.4 | C |
| 18 | $U(O_3PC_6H_5)_2$ | 2.04 | $H_2O_3PC_6H_5$ | 1.29 | $UCl_4$ 1.55 | 26.06 | 1.97 | | 41.8 | 15 | C |
| 19 | $Ti(O_3PCH{=}CH_2)_2$ | 7.1 | $Na_2O_3P$ $-CH{=}CH_2$ | 9.44 | $TiCl_4$ 16.3 | | | | | | |
| 20 | $Th(O_3PCH{=}CH_2)_2$ | 7.9 | $Na_2O_3P$ $-CH{=}CH_2$ | 10.6 | $Th(NO_3)_4$ 21.0 | | | | | | |
| 21 | $Th(O_3PCH_2CH_2COOH)_2$ | | | | | | | | | | |
| 22 | $Ti(O_3PC_6H_5)_2$ | 5.722 | $H_2O_3PC_6H_5$ | 5.665 | $TiOCl_2$ 8.087 | | | | | | |
| 23 | $Th(p\text{-}O_3POC_6H_4NO_2)_2$ | 2.763 | $Na_2PO_4C_6H_4$ $-NO_2$ | 3.032 | $Th(NO_3)_4$ 2.209 | 18.54 | 2.11 | 2.56 N | | 16.4 | S |
| 24 | $Ce(O_3PC_6H_5)_2$ | | $H_2O_3PC_6H_5$ | | $H_4Ce(SO_4)_4$ | 32.56 | 2.52 | | 25.3 | 15.5 | C |

TABLE III (Continued)

| Ex. | Compound Produced | Product Wt. g | Phosphorus Reagent Used g. | Acid Wt. g | $M^{+4}$ Salt Wt. g | Weight Percent | | | | I.S. A | Str. |
|---|---|---|---|---|---|---|---|---|---|---|---|
| | | | | | | C | H | Other | $M^{+4}$ | | |
| 25 | $Ti(O_3PCH_3)_2$ | 0.919 | $H_2O_3PCH_3$ | 0.913 | $TiOCl_2$ 0.640 | 6.54 | 4.54 | 17.5 P | | | A |
| 26 | $Th(O_3PCH_3)_2$ | 1.704 | $H_2O_3PCH_3$ | 0.845 | $Th(NO_3)_4$ 2.252 | 4.78 | 2.21 | | 51.4 | 8.92 | C |
| 27 | $U_{1/2}Ce_{1/2}(p\text{-}O_3PC_6H_4OCH_3)_2$ | 0.648 | $H_2O_3PC_6H_4$ $-OCH_3$ | 0.800 | $H_4Ce(SO_4)_4$ 3 $UCl_4$ 0.404 | | | | | | |

Examples 28—51

Using the method outlined in Example 1, the following compounds are prepared:

| Ex. | | |
|---|---|---|
| 28 | $M(O_3P-(CH_2)_n-PR_2)_2$ | M = $Ti^{+4}$, $Hf^{+4}$, $U^{+4}$, $Th^{+4}$, $Ce^{+4}$, $Pb^{+4}$; n = 1–10; R = $-CH_3$, $-C_2H_5$, $-C_6H_5$. |
| 29 | $M(O_3P-(CH_2)_n-CH_3)_2$ | M as above and n = 1–22. |
| 30 | $M(O_3P-(CH_2)_n-OP(OR)_2)_2$ | M, n, R as above. |
| 31 | $M(O_3P-(CH_2)_n-\overset{+}{N}(CH_3)_3X^-)_2$ | M, n as above; X = halide, sulfate nitrate, phosphate, acetate. |
| 32 | $M(O_3P-C_6H_4X)_2$ | M as above. X = halogen, sulfo, nitro phosphono, acetoxy |
| 33 | $M(O_3P-CH_2-C_6H_4X)_2$ | M and X as in Ex. 32 |
| 34 | $M(O_3P-(CH_2)_n-NH-CS_2H)_2$ | M, n as above. |
| 35 | $M(O_3P(CH_2)_n-N(CH_2CO_2H)_2$ | M, n as above. |
| 36 | $M(O_3P(CH_2)_n-\overset{+}{N}H_2-(CH_2)_3SO_3^-)_2$ | M, n as above. |
| 37 | $M(O_3P-(CH_2)_n-NC)_2$ | M, n as above. |
| 38 | $M(O_3P-(CH_2)_n-(C\equiv CH)_2$ | M, n as above. |
| 39 | 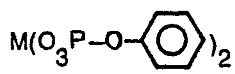 | M as above. |
| 40 | 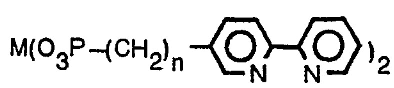 | M, n as above. |
| 41 | $M(O_3P-(CH_2)_n-SR)_2$ | M, n as above. R = $-CH_3$, $-C_2H_5$. |
| 42 | 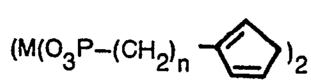 | M, n as above. |
| 43 | $(M(O_3P-(CH_2)_n\overset{O}{\overset{\Vert}{C}}H)_2$ | M, n as above. |
| 44 | 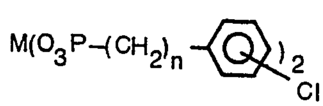 | M, n as above. |
| 45 | 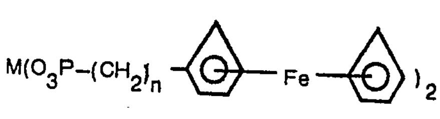 | M, n as above. |

| Ex. | | |
|---|---|---|
| 46 | $M(O_3P{-}(CH_2)_n{-}C(SH){=}CH(SH))_2$ | M, n as above. |
| 47 | 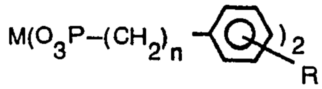 | M, n as above, $R = -CH_3$, $-C_2H_5$, $-CH(CH_3)_2$ or $-C(CH_3)_3$. |
| 48 | $M(O_3P{-}(CH_2)_nOPR_2)_2$ | M, n and R as in 1. |
| 49 | $M(O_3P{-}(CH_2)_n{-}Br)_2$ | M, n as above. |
| 50 | $M(O_3P{-}(CF_2)_n{-}SO_3H)_2$ | M, n as above. |
| 51 | Compounds above in which the $P{-}(CH_2)_n$ linkage is replaced by a $P{-}O{-}(CH_2)_n$ link. | |

## Example 52

An aqueous solution of organic compounds for use in absorption experiments was prepared by shaking 25 ml of chloroform, 25 ml of benzene and 25 ml of n-hexanol in a separatory funnel with 100 ml of deionized water. After settling, the aqueous layer was separated and placed into a 125 ml Erlenmeyer flask containing a stirring bar and the flask was sealed. This solution was stirred continuously during sampling and experimentation. First, some small samples were taken about every 15 minutes to establish a baseline concentration of each organic in the water.

A 2.442 g sample of ground

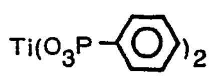

was added quickly to the solution. This mixture was shaken for a minute to prevent the solid from floating on the water. Aqueous samples were taken after allowing the solid to partially settle in order to obtain a clear solution for gas chromatographic analysis.

On gas chromatograph testing, a slow but continuous drop of organics and then a sudden drop in concentration of all three (n-hexanol, benzene and chloroform) was shown when the

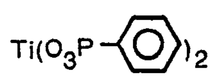

was added to the system. A leveling off occurred after the initial rapid extraction and at time infinity.

A plot of the area of an ethanol peak from gas chromatography data shows a fairly constant concentration of the ethanol throughout the whole experiment indicating it is a reliable internal reference peak. Ethanol seemed more soluble in water over the solid. The ethanol came from the chloroform used in the experiment. The chloroform contained about one percent ethanol stabilizer. The

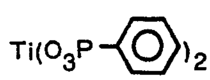

had an affinity to absorb the organic compounds. The weight distribution coefficient ($K_{wt}$) and the molar distribution coefficient ($K_m$) for the n-hexanol, chloroform and benzene were determined to be as follows for the

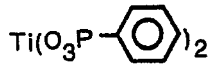

| | | |
|---|---|---|
| N-hexanol | $K_{wt}$ 28.22 | $K_m$ 564.2 |
| Chloroform | 20.26 | 405.1 |
| Benzene | 53.19 | 1063.5 |

These results indicate the utility of the compound as a selective sorbent which can remove specific contaminants from solutions while leaving other dissolved materials in the solution.

Example 53

Titanium phenylphosphonate having a surface area of about 151—167 $m^2/g$ was evaluated as sorption solid. A sample of water was contaminated with 1-hexanol (1700 ppm), chloroform (1100 ppm) and benzene (300 ppm).One hundred ml of this solution was treated with 2.4 g of the titanium phenylphosphonate. Analysis established that the solid absorbed the organics. The distribution coefficients were 750 (benzene), 430 (1-hexanol) and 250 ($CHCl_3$). Absorption of benzene was preferred.

The following Test Procedures 1—15 are illustrative of simple utility screening test procedures which can be used to show ways of using the solid compounds of the invention whether crystalline, semicrystalline, or amorphous. Of course, the tests can be modified by the skilled chemist to suit the fundamental character of the compound being screened.

Test Procedure 1

The usefulness of a tetravalent element 2-carboxyethyl phosphonate was shown in an experiment which tested the ability of the compound to extract copper ions from aqueous solutions.

In the experiment, 1.00 g of the tetravalent element 2-carboxyethyl phosphonate was mixed with 40 ml of 0.103 M copper solution having a pH of 4.01 and, after about 30 minutes, a 10 ml aliquot of the solution phase was removed, its pH being 2.19. The remaining slurry was treated with 4 ml of 2.5 percent of sodium hydroxide solution and, after about 10 minutes, the liquid, with a pH of 3.93, was removed. A second 1.11 g portion of the tetravalent metal 2-carboxyethyl phosphonate was mixed with 40 ml of the 0.103 M copper solution, and 2.0 ml of 2.5 percent sodium hydroxide solution was added after about 15 minutes a 10 ml aliquot of the supernatant liquid, which has a pH of 3.39, was removed. The remaining slurry was treated with 5.0 ml of 2.5 percent sodium hydroxide solution and after about 30 minutes, the supernatant liquid having a pH of 4.85 was removed.

A loading curve was prepared by plotting the pH of the solution versus the milli equivalents of copper extracted per gram of tetravalent element 2-carboxyethyl phosphonate.

Test Procedure 2

The ion exchange capability was demonstrated for both the sulfonic acid and sodium sulfonate forms a tetravalent element 3-sulfopropyl phosphonate.

A 0.50 g portion of the acid form was slurried with 10 ml of 0.215 N copper sulfate solution. The pH of the solution was initially 3.80 but immediately dropped to 0.92, the intially white solid became a pale blue color, and the blue solution color decreased markedly in intensity. Atomic absorption analysis of the solution after exchange indicated a copper concentration of 0.093 N, for copper loading in the solid of 2.46 meq/g, or 77 percent of the theoretical capacity.

The exchange experiment was repeated with the sodium sulfonate form of the compound. After exchange, the solution had a pH of 2.88 and a copper content of 0.135 N. Loading of the solid was calculated as 1.62 meq/g, or 51 percent of the theoretical cpacity.

Test Procedure 3

The mixed component product of $M^{+4}(O_3P(CH_2)_{10}PO_3)_{4/3}(O_3POH)_{2/3}$ was shown to be very selective in its complexative absorption of amines by virtue of the ten carbon cross-links from one layer to the next. This behaviour is a form of "molecular sieving."

In four separate experiments the behavior of two —OH containing tetravalent metal layered solid toward two different amines was investigated. The two amines were a bulky trioctylamine and a small ethylamine. As the table below indicates, the noncross-linked tetravalent metal phosphate picked up both amines from a methanolic solution. However, the mixed component product picked up only the small amine, due to the constricting effect of the bridging ten carbon group.

ABSORPTION OF AMINES

| Solid | Amine | Molar Ratio of Amine/—OH Group in Product |
|---|---|---|
| $M(O_3P—OH)_2$ | $C_2H_5NH_2$ | 0.86 |
| $M(O_3P—OH)_2$ | $(C_8H_{17})_3N$ | 0.24 |
| $M(O_3P(CH_2)_{10}PO_3)_{1/3}(O_3POH)_{4/3}$ | $C_2H_5NH_2$ | 0.31 |
| $M(O_3P(CH_2)_{10}PO_3)_{1/3}(O_3POH)_{4/3}$ | $(C_8H_{17})_3N$ | 0.00 |

Test Procedure 4

Extraction of palladium +2 ion from aqueous solution by ion exchange with

$$M(O_3P\phi)_{8/5}(O_3PCH_2CH_2CO_2H)_{2/5}.$$

A solution of palladium (II) chloride was prepared by dissolving about 1.0 g of commercially available palladium (II) chloride in about 100 ml of water under a nitrogen purge. A small amount of undissolved material was removed by filtration. The pH of this solution was 2.90. To this solution was added 3.0 g of

$$M(O_3P\phi)_{8/5}(O_3PCH_2CH_2CO_2H)_{2/5}.$$

The pH decreased to 2.35. Using an auto-titrator in a pH stat mode, the pH was raised in small steps to 3.5 by addition of 0.10 N aqueous sodium hydroxide. The pale yellow solid product was isolated by filtration and washed successively wth water, acetone and ethyl ether. After oven drying, elemental analysis indicated 3.72% Pd content of the solid phase.

This example demonstrates the extraction of a precious metal, more broadly a Group VIII metal, from solution. The palladium-containing product incorporates a catalytically active species and represents a novel example of a heterogenized or anchored catalyst which can be used for the reactions shown in the Bailar and Hartley & Vezey publications incorporated herein.

Test Procedure 5

Esterification of tetravalent element 3-carboxypropyl phosphonate with n-butanol.

To 100 ml three-necked flask was charged 5.0 g of tetravalent metal 3-carboxypropylphosphonate, 40 g n-butanol and 10 g $H_2O$. To this was added 3 ml of HCl as catalyst. The slurry was refluxed and water removed azeotropically. After about one day, 40 ml of fresh butanol was added and azeotropic distillation continued for about a week. The product was isolated by filtration and washed with acetone and ethyl ether. The dry product weighed 5.44 g. The infrared spectrum clearly shows the conversion from the carboxylic acid to the ester. This material can be used as a host or carrier for biologically active organic molecules (e.g., methoprene).

Test Procedure 6

The composed tetravalent element 3-sulfopropyl phosphonate was used as a catalyst in an esterification reaction. A 0.503 g portion was added to a distillation flask containing 2.85 ml of acetic acid and 2.85 ml of denatured ethanol. The mixture was heated and a distillate product collected. This product was identified by gas chromatography and infrared spectrophotometry as ethyl acetate.

The solid phase of the reaction mixture was recovered and weighed 0.51 g. Its X-ray diffraction pattern matched that of the initial material added.

Test Procedure 7

A slurry of 0.100 g of tetravalent metal 3-sulfopropyl phosphonate and 1.0 g cyclohexanol was heated to 125°C in a micro distillation apparatus. An essentially quantitative yield of cyclohexene was recovered in the distillate receiver, indicating utility of the tetravalent metal 3-sulfopropylphosphonate as a catalyst for dehydrating alcohols.

Test Procedure 8

Diethyl 2-carboethoxyethyl phosphonate was prepared by the Arbuzov reaction of triethyl phosphite and ethyl 3-bromopropionate. The phosphonate ester product was hydrolyzed to the acid in refluxing HBr and the reacted in situ with a tetravalent element ion. The resultant layered compound, tetravalent metal 2-carboxyethyl -phosphonate, had interlamellar carboxylic acid substituents. The highly crystalline modification had an interlayer distance of 12.8Å and its n-hexylammonium salt was determined to have interlayer distance of 27.2Å. Thorium 2-carboxyethyl phosphonate was also prepared in an analogous manner.

The interlamellar carboxylic acid was determined to have a strong carbonyl stretching frequency at 1710 $cm^{-1}$. Upon sodium salt formation this shifts to 1575 and 1465 $cm^{-1}$. The X-ray powder diffraction pattern of the sodium salt indicates a layer spacing of 14.2Å. The X-ray and infra-red data of the interlamellar carboxylic acid and its salts indicate that this material behaves as a carboxylic acid. This IR behavior is analogous to that of ion exchange resins with carboxylic functionality.

The ion exchange behavior of the interlamellar carboxylic acid was investigated with a number of elements. The $pH_{0.5}$ is about 3.8 for the semi-crystalline and about 4.5 for the highly crystalline. This indicates that the matrix supporting the anchored functional group influences the reactivity of the functional group.

The interlamellar ion also has an influence on the $H^+/Cu^{+2}$ exchange equilibrium. High crystallinity modifications of thorium and zirconium 2-carboxyethylphosphonate were compared. The thorium compound is the stronger acid by about 0.3 pKa units in this reaction ($pH_{0.5} = 4.2$ vs 4.5).

Test Procedure 9

The reaction rate of tetravalent element 2-carboxyethylphosphonate with aqueous sodium hydroxide was determined by its addition to an aqueous solution of NaOH with decrease in pH measured as a function of time. The concentration of hydroxide ion changed by over three orders of magnitude in 15 seconds representing reaction of 80% of the carboxylic groups. This established that the interlamellar reaction was quite facile and diffusion into the crystal did not involve a high kinetic barrier. Prolonged exposure at a pH of about 9 to 10 or higher, however, resulted in hydrolysis of the crystal with formation of the metal oxide.

Test Procedure 10

Solid tetravalent element 2-bromethyl phosphonate was slurried in an aqueous solution of 2-carboxyethyl phosphonic acid. A trace (1% mol) of HF was added and the mixture refluxed overnight. The infrared spectrum of the solid after this period definitely showed the presence of the carboxylic acid carbonyl band at 1710 $cm^{-1}$.The X-ray powder pattern of the exchanged product was virtually identical to the starting material. This was likely due to the fact that tetravalent element 2-bromethyl phosphonate has an interlayer spacing of 13.0Å and the 2-carboxy analog 12.8Å. Based on stoichiometry, about 5 to 10% of the sites were exchanged. This being more than the apparent surface site, interlamellar exchange took place.

Test Procedure 11

In an experiment to determine the ability of tetravalent element bis(mercaptomethylphosphonate) to extract silver ions from solution, 0.076 g of the compound was shaken in a vial with 5 ml of 0.10 M silver nitrate. The mixture was allowed to stand for several days, after which a sample of the supernatant liquid was decanted for analysis.

The original silver solution contained 10.8 g/l silver and the "extracted" solution was found to contain 4.96 g/l silver, indicating that 5.85 g/l silver was extracted by the compound.

Test Procedure 12

An experiment was performed to determine the ability of a layered cyano end terminated polymer to extract copper ions from aqueous solution. A 0.09 g portion of the tetravalent element bis(2-cyanoethylphosphate) compound was mixed with 5 ml of a solution containing 0.1M $CuSO_4$, 0.5M $CH_3COONa$ and 0.5M $CH_3COOH$. The mixture was permitted to stand for a day and a portion of the supernatant liquid was decanted and analyzed for copper.

The initial copper solution contained 6.45 g/l cu and the "extracted" solution contained 5.94 g/l Cu, indicating an extraction of 0.51 g/l copper.

Test Procedure 13

The experiment of Procedure 12 was repeated using 0.091 g of the compound and 5 ml of the unbuffered 0.1M $CuSO_4$ solution.

Analyses of the initial copper solution gave a value of 6.33 g/l Cu, and the "extracted" solution contained 5.89 g/l Cu, indicating an extraction of 0.44 g/l copper.

Test Procedure 14

An experiment to determine the ability of tetravalent element bis(2-cyanoethylphosphate) to extract silver ions from aqueous solution was performed. A 0.090 g portion of the compound was mixed with 5 ml of 0.1M $AgNO_3$ solution, allowed to stand for several days and a portion of the liquid decanted for analysis.

The initial silver solution contained 10.8 g/l Ag and the "extracted" solution contained 9.82 g/l Ag showing an extraction of 0.98 g/l Ag.

No claim is made herein to anything that is claimed in co-pending Application No. 79301989.4 (EP—A—0,010,857) filed on the same date as the present Application.

**Claims for the Contracting States: BE CH DE FR GB IT NL SE**

1. A solid inorganic polymer having basic structural units of the formula:

$$M(O_3PO_xR)_n$$

in which M is one or more of the tetravalent elements tungsten, uranium, titanium, thorium, tellurium, tin, silicon, rubidium, plutonium, praseodymium, lead, osmium, niobium, molybdenum, manganese, iridium, hafnium, germanium and cerium; R is one or more organo, acyclic, alicyclic, heterocyclic, heterocyclic or aromatic groups, x is 0 or 1; and n is 1 or 2, provided that when n is 1, R is terminated with an unsubstituted phosphate or phosphonate group and contains at least two carbon atoms separating the phosphorus atoms.

2. A solid inorganic polymer having basic structural units of the formula:

$$M(O_3PO_xR)_n$$

in which M is one or more of the tetravalent metals titanium, molybdenum, tin, cerium, hafnium, lead, thorium and uranium; R is one or more organo acyclic, alicyclic, heteroacyclic, heterocyclic or aromatic groups; x is 0 or 1; and n is 1 or 2, provided that when n is 1, R is terminated with an unsubstituted phosphate or phosphonate group and contains at least two carbon atoms separating the phosphorus atoms.

3. A polymer, as claimed in claim 1 or 2, in which at least one R is an acyclic group consisting of a straight chain or branched alkyl, alkenyl or alkynyl group, either unsubstituted or substituted with one or more of thio, halo, oxo, hydroxy, carbonyl, carboxy, alkylcarbonyloxy, mercapto, sulfinyl, sulfonyl, imino, amino, cyano, nitro, hydroxyamino, nitroso, cycloalkyl, aryl, aralkyl, aryloxy, arylalkoxy, arylthio, aralkylthio, arylamino, or aralkylamino.

4. A polymer, as claimed in claim 1 or 2, in which at least one R is an alicyclic group consisting of a saturated or unsaturated cyclic aliphatic group, either unsubstituted or substituted with one or more of alkyl, alkenyl, alkynyl, alkoxy, alkenyloxy, alkynyloxy, alkylthio, alkenylthio, alkynylthio, halo, oxo, hydroxy, carbonyl, carboxy, alkylcarbonyloxy, alkylcarboxyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino or alkarylamino.

5. A polymer, as claimed in claim 1 or 2, in which at least one R is an aromatic group consisting of a phenyl, naphthyl, biphenyl, anthracyl or phenanthryl group, either unsubstituted or substituted with one or more of alkyl, alkenyl, alkynyl, alkoxy, alkenyloxy, alkynyloxy, alkylthio, alkenylthio, alkynylthio, halo, hydroxy, carbonyl, carboxy, alkylcarbonyloxy, alkylcarbonyl, carboxyalkyl, thio, mercapto, sulfinyl,, sulfonyl, imino, amino, cyano, nitro, hydroxyamino, nitroso, cycloalkyl, cycloalkaryl, alkaryl, alkaryloxy, alkarylthio, or alkarylamino.

6. A polymer, as claimed in claim 1 or 2, in which at least one R is a heterocyclic group consisting of an alicyclic or aromatic group containing one or more of the heteroatoms oxygen, nitrogen or sulfur in the ring, which group can be substituted with one or more of alkyl, alkenyl, alkynyl, alkoxy, alkenyloxy, alkynyloxy, alkylthio, alkenylthio, alkynylthio, halo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino, and alkarylamino.

7. A polymer, as claimed in claim 1 or 2, in which at least one R is a heteroacyclic group consisting of a branched or straight chain, saturated or unsaturated, acyclic group containing one or more of the heteroatoms oxygen, nitrogen or sulfur in the chain, which group can be substituted with one or more of halo, hydroxy, carbonyl, mercapto, sulfinyl, sulfonyl, imino, amino, cyano, nitro, hydroxyamino, nitroso, aryl, aryloxy, arylthio, and arylamino.

8. A polymer, as claimed in claim 1 or 2, having basic structural units of the formula:

$$M(O_3PO_xRCOOH)_2.$$

and in which M, x and R have the same significance as in claims 1 and 2.

9. A polymer, as claimed in claim 1 or 2, having basic structural units of the formula:

$$M(O_3PO_xRSO_3H)_2$$

and in which M, x and R have the same significance as in claims 1 and 2.

10. A polymer, as claimed in claim 1 or 2, having basic structural units of the formula:

$$M(O_3PO_xRCN)_2$$

and in which M, x and R have the same significance as in claims 1 and 2.

11. A polymer, as claimed in claim 1 or 2, having basic structural units of the formula:

$$M(O_3PO_xRSH)_2$$

and in which M, x and R have the same significance as in claims 1 and 2.

12. A polymer, as claimed in claim 6 or 7, wherein a heteroatom is sulfur.

13. A polymer, as claimed in claim 6 or 7, wherein a heteroatom is oxygen.

14. A polymer, as claimed in claim 6 or 7, wherein a heteroatom is nitrogen.

15. A polymer, as claimed in claim 3, providing layer-bridging organo groups containing structural units of the formula:

$$M(O_3PO_xRO_xPO_3)$$

and in which M, x and R have the same significance as in claim 3.

16. A polymer, as claimed in claim 1, 2, 8, 9, 10 or 11, further comprising from about 0.01 to about 10 percent by weight of a Group VIII metal.

17. A polymer, as claimed in claim 16, wherein a Group VIII metal is palladium.

18. The use of a polymer as claimed in any preceding claim for catalysis.

19. The use of a polymer as claimed in any one of claims 1 to 17 for sorption of organic compounds.

20. The use of a polymer as claimed in any one of claims 1 to 17 for ion exchange.

21 The use of a polymer as claimed in any one of claims 1 to 17 for ion complexing.

22. The use of a polymer as claimed in any one of claims 1 to 17 for formulating controlled release compositions.

23. The use of a polymer as claimed in any one of claims 1 to 17 as an additive to polymeric compositions.

**Claims for the contracting state: AT**

1. A method for preparing a new inorganic polymer, characterised in that said polymer has basic structural units of the formula:

$$M(O_3PO_xR)_n$$

and in that for M one or more of the tetravalent elements tungsten, uranium, titanium, thorium, tellurium, tin, silicon, rubidium, plutonium, praseodymium, lead, osmium, niobium, molybdenum, manganese, iridium, hafnium, germanium and cerium is used; for R one or more organo, acyclic, alicyclic, heteroacyclic, heterocyclic or aromatic groups is used; X is chosen to be 0 or 1; and n is chosen to be 1 or 2, provided that when n is 1, R is terminated with an unsubstituted phosphate or phosphonate group and contains at least two carbon atoms separating the phosphorus atoms.

2. A method for preparing a new solid inorganic polymer, characterised in that said polymer has basic structural units of the formula:

$$M(O_3PO_xR)_n$$

and in that for M one or more of the tetravalent metals titanium, molybdenum, tin, cerium, hafnium, lead, thorium and uranium is used; for R one o- more organo acyclic, alicyclic, heteroacyclic, heterocyclic or aromatic groups is used; x is chosen to be 0 or 1; and n is chosen to be 1 or 2, provided that when n is 1, R is terminated with an unsubstituted phosphate or phosphonate group and contains at least two carbon atoms separating the phosphorus atoms.

3. A method as claimed in claim 1 or 2, in which an acyclic group consisting of a straight chain or branched alkyl, alkenyl or alkynyl group, either unsubstituted or substituted with one or more of thio, halo, oxo, hydroxy, carbonyl, carboxy, alkylcarbonyloxy, mercapto, sulfinyl, sulfonyl, imino, amino, cyano, nitro, hydroxyamino, nitroso, cycloalkyl, aryl, aralkyl, aryloxy, arylalkoxy, arylthio, aralkylthio, arylamino, or aralkylamino, is used for at least one R.

4. A method as claimed in claim 1 or 2, in which an alicyclic group consisting of a saturated or unsaturated cyclic aliphatic group, either unsubstituted or substituted with one or more of alkyl, alkenyl, alkynyl, alkoxy, alkenyloxy, alkynyloxy, alkylthio, alkenylthio, alkynylthio, halo, oxo, hydroxy, carbonyl, carboxy, alkylcarbonyloxy, alkylcarboxyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino or alkarylamino, is used for at least one R.

5. A method as claimed in claim 1 or 2, in which an aromatic group consisting of a phenyl, naphthyl, biphenyl, anthracyl or phenanthryl group, either unsubstituted or substituted with one or more of alkyl, alkenyl, alkynyl, alkoxy, alkenyloxy, alkynyloxy, alkylthio, alkenylthio, alkynylthio, halo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, cycloalkyl, cycloalkaryl, alkaryl, alkaryloxy, alkarylthio, or alkarylamino, is used for at least one R.

6. A method as claimed in claim 1 or 2, in which a heterocyclic group consisting of an alicyclic or aromatic group containing one or more of the heteroatoms oxygen, nitrogen or sulfur in the ring, which group can be substituted with one or more of alkyl, alkenyl, alkynyl, alkoxy, alkenyloxy, alkynyloxy, alkylthio, alkenylthio,. alkynylthio, halo, hydroxy, carbonyl, carboxy, alkylcarbonyloxy, alkylcarbonyl, carboxyalkyl, thio, mercapto, sulfinyl, sulfonyl, imino, amino, cyano, nitro, hydroxyamino, nitroso, aryl, aralkyl, alkaryl, aryloxy, arylalkoxy, alkaryloxy, arylthio, aralkylthio, alkarylthio, arylamino, aralkylamino, and alkarylamino, is used for at least one R.

7. A method as claimed in claim 1 or 2, in which a heteroacyclic group consisting of a branched or straight chain, saturated or unsaturated, acyclic group containing one or more of the heteroatoms oxygen, nitrogen or sulfur in the chain, which group can be substituted with one or more of halo, hydroxy, carbonyl, mercapto, sulfinyl, sulfonyl, imino, amino, cyano, nitro, hydroxyamino, nitroso, aryl aryloxy, arylthio, and arylamino, is used for at least one R.

8. A method as claimed in claim 1 or 2, characterised in that the polymer has basic structural units of the formula:

$$M(O_3PO_xRCOOH)_2.$$

and in which M, x and R have the same significance as in claims 1 and 2.

9. A method as claimed in claim 1 or 2, characterised in that the polymer has basic structural units of the formula:

$$M(O_3PO_xRSO_3H)_2$$

and in which M, x and R have the same significance as in claims 1 and 2.

10. A method as claimed in claim 1 or 2, characterised in that the polymer has basic structural units of the formula:

$$M(O_3PO_xRCN)_2$$

and in which M, x and R have the same significance as in claims 1 and 2.

11. A method as claimed in claim 1 or 2, characterised in that the polymer has basic structural units of the formula:

$$M(O_3PO_xRSH)_2$$

and in which M, x and R have the same significance as in claims 1 and 2.

12. A method as claimed in claim 6 or 7, wherein sulfur is used as a heteroatom.

13. A method as claimed in claim 6 or 7, wherein oxygen is used as a heteroatom.

14. A method as claimed in claim 6 or 7, wherein nitrogen is used as a heteroatom.

15. A method as claimed in claim 3, characterised in that layer-bridging organo groups containing structural units of the formula:

$$M(O_3PO_xRO_xPO_3)$$

and in which M, x and R have the same significance as in claim 3, are provided in the polymer.

16. A method as claimed in claim 1, 2, 8, 9, 10 or 11, characterised in that from about 0.01 to about 10 percent by weight of a Group VIII metal is provided in the polymer.

17. A method as claimed in claim 16, wherein palladium is used as a Group VIII metal.

18. The use for catalysis of a polymer prepared by a method as claimed in any preceding claim.

19. The use for sorption of organic compounds of a polymer prepared by a method as claimed in any one of claims 1 to 17.

20. The use for ion exchange of a polymer prepared by a method as claimed in any one of claims 1 to 17.

21. The use for ion complexing of a polymer prepared by a method as claimed in any one of claims 1 to 17.

22. The use for formulating controlled release compositions of a polymer prepared by a method as claimed in any one of claims 1 to 17.

23. The use as an additive to polymeric compositions of a polymer prepared by a method as claimed in any one of claims 1 to 17.

**Revendications pour les etats contractants: BE CH DE FR GB IT NL SE**

1. Polymère minéral nouveau caractérise en ce qu'il présente des motifs structuraux fondamentaux de formule:

$$M(O_3PO_xR)_n$$

et en ce que:

M est au moins un élément tétravalent consistant en tungstène, uranium, titane, thorium, tellure, étain, silicium, rubidium, plutonium, praseodyme, plomb, osmium, niobium, molybdène, manganèse, iridium, hafnium, germanium et cerium,

R est un ou plusieurs des groupes organoacycliques, alicycliques, hétéroacycliques, hétérocycliques ou aromatiques,

x est égal à 0 ou 1,

n est égal à 1 ou 2, à condition que lorsque n est égal à 1, R se termine par un groupe phosphate ou phosphonate non substitué et contienne au moins 2 atomes de carbone séparant les atomes de phosphore.

2. Polymère minéral solide nouveau, caractérisé en ce qu'il présente des motifs structuraux fondamentaux de formule:

$$M(O_3PO_xR)_n$$

et en ce que:

M est au moins un des metaux tétravalents: titane, molybdène, étain, cerium, hafnium, plomb, thorium ou uranium,

R est un ou plusieurs des groupes organoacycliques, alicycliques, hétéroacycliques, hétérocycliques ou aromatiques,

x est égal à 0 ou 1,

n est égal à 1 ou 2, à condition que lorsque n est égal à 1, R se termine par un groupe phosphate ou phosphonate non substitué et contienne au moins 2 atomes de carbone séparant les atomes de phosphore.

3. Polymère selon la revendication 1 ou 2, caractérisé en ce qu'au moins un des groupes R est un radical acyclique consitant en un groupe alkyle, alkényle ou alkynyle à chaine linéaire ou ramifiée, soit non substitué soit substitué par un ou plusieurs des groupes thio, halo, oxo, hydroxy, carbonyle, carboxy, alkylcarbonyloxy, mercapto, sulfinyle, sulfonyle, imino, amino, cyano, nitro, hydroxyamino, nitroso, cycloalkyle, aryle, aralkyle, aryloxy, arylalkoxy, arylthio, aralkylthio, arylamino ou aralkylamino.

4. Polymère selon la revendication 1 ou 2, caractérisé en ce qu'au moins un des groupes R est un radical alicyclique consistant en un groupe aliphatique cyclique saturé ou insaturé, soit non-substitué soit substitué avec un ou plusieurs des substituants de la liste formée par les radicaux alkyle, alkényle, alkynyle, alkoxy, alkényloxy, alkynyloxy, alkylthio, alkénylthio, alkynylthio, halo, oxo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino ou alkarylamino.

5. Polymère selon la revendication 1 ou 2, caractérisé en ce qu'au moins un des groupes R est un radical aromatique consistant en un groupe phényle, naphtyle, biphényle, anthracyle ou phénantryle, soit non substitué soit substitué par un ou plusieurs des groupes alkyle, alkényle, alkynyle, alkoxy, alkényloxy, alkynyloxy, alkylthio, alkénylthio, alkynylthio, halo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, cycloalkyle, cycloalkaryle, alkaryle, alkaryloxy, alkarylthio ou alkarylamino.

6. Polymère selon la revendication 1 ou 2, caractérisé en ce qu'au moins un des groupes R est un radical hétérocyclique consistant en un groupe alicyclique ou aromatique contenant un ou plusieurs hétéroatomes consistant en oxygène, azote ou soufre dans le noyau, ce groupe pouvant être substitué par un ou plusieurs substituants consistant en les radicaux alkyle, alkényle, alkynyle, alkoxy, alkényloxy, alkynyloxy, alkylthio, alkénylthio, alkynylthio, halo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino et alkarylamino.

7. Polymère selon la revendication 1, caractérisé en ce qu'au moins un des groupes R est un groupe hétérocyclique consistant en un radical acyclique à chaine linéaire ou ramifiée, saturé ou insaturé, contenant un ou plusieurs hétéroatomes consistant en oxygène, azote ou soufre dans le noyau, ce groupe pouvant être substitué par un ou plusieurs substituants consistant en les radicaux halo, hydroxy, carbonyle, mercapto, sulfinyle, sulfonyle, imino, amino, cyano, nitro, hydroxyamino, nitroso, aryle, aryloxy, arylthio et arylamino.

8. Polymère selon la revendication 1 ou 2, caractérisé en ce qu'il comprend des motifs structuraux fondamentaux de formule:

$$M(O_3PO_xRCOOH)_2$$

dans laquelle M, x et R ont les mêmes significations que dans les revendications 1 et 2.

9. Polymère selon la revendication 1 ou 2, caractérisé en ce qu'il comprend des motifs structuraux fondamentaux de formule:

$$M(O_3PO_xRSO_3H)_2$$

dans laquelle M, x et R ont les mêmes significations que dans les revendications 1 et 2.

10. Polymère selon la revendication 1 ou 2, caractérisé en ce qu'il comprend des motifs structuraux fondamentaux de formule:

$$M(O_3PO_xRCN)_2$$

dans laquelle M, x et R ont les mêmes significations que dans les revendications 1 et 2.

11. Polymère selon la revendication 1 ou 2, caractérisé en ce qu'il comprend des motifs structuraux fondamentaux de formule:

$$M(O_3PO_xRSH)_2$$

dans laquelle M, x et R ont les mêmes significations que dans les revendications 1 et 2.

12. Polymère selon la revendication 6 ou 7, caractérisé en ce que l'hétéroatome est le soufre.

13. Polymère selon la revendication 6 ou 7, caractérisé en ce que l'hétéroatome est l'oxygène.

14. Polymère selon la revendication 6 ou 7, caractérisé en ce que l'hétéroatome est l'azote.

15. Polymère selon la revendication 3, caractérisé en ce qu'il comprend des groupes organiques de pontage entre couches contenant des motifs structuraux de formule:

$$M(O_3PO_xRO_xPO_3)$$

et dans laquelle M, x et R ont les mêmes significations que dans les revendications 1 et 2.

16. Polymère selon la revendication 1, 2, 8, 9, 10 ou 11 caractérisé en ce qu'il contient en outre 0,01 à 10% en poids d'un métal de groupe VIII.

17. Polymère selon la revendication 16, dans lequel le métal du groupe VIII est le palladium.

18. Application des polymères selon l'une quelconque des revendications précédentes à la catalyse.

19. Application des polymères selon l'une quelconque des revendications 1 à 17 pour la sorbtion de composés organiques.

20. Application des polymères selon l'une quelconque des revendications 1 à 17 pour l'échange ionique.

21. Application des polymères selon l'une quelconque des revendications 1 à 17 pour la complexation ionique.

22. Application des polymères selon l'une quelconque des revendications 1 à 17 pour la formulation de compositions à libération contrôlée.

23. Application des polymères selon l'une quelconque des revendications 1 à en tant qu'additifs de compositions polymères.

**Revendications pour l'etat contractant: AT**

1. Procédé de préparation d'un polymère minéral nouveau caractérisé en ce que ledit polymère présente des motifs structuraux fondamentaux de formule:

$$M(O_3PO_xR)_n$$

et en ce que:

M est au moins un élément tétravalent consistant en tungstène, uranium, titane, thorium, tellure, étain, silicium, rubidium, plutonium, praseodyme, plomb, osmium, niobium, molybdène, manganèse, iridium, hafnium, germanium et cerium,

R est un ou plusieurs des groupes organoacycliques, alicycliques, hétéroacycliques, hétérocycliques ou aromatiques,

x est égal à 0 ou 1,

n est égal à 1 ou 2, à condition que lorsque n est égal à 1, R se termine par un groupe phosphate ou phosphonate non substitué et contienne au moins 2 atomes de carbone séparant les atomes de phosphore.

2. Procédé de préparation d'un polymère minéral solide nouveau, caractérisé en ce que ledit polymère présente des motifs structuraux fondamentaux de formule:

$$M(O_3PO_xR)_n$$

et en ce que:

M est au moins un des métaux tétravalents: titane, molybdène, étain, cerium, hafnium, plomb, thorium ou uranium,

R est un ou plusieurs des groupes organocycliques, alicycliques, hétéroacycliques, hétérocycliques ou aromatiques,

x est égal à 0 ou 1,

n est égal à 1 ou 2, à condition que lorsque n est égal à 1, R se termine par un groupe phosphate ou phosphonate non substitué et contienne au moins 2 atomes de carbone séparant les atomes de phosphore.

3. Procédé selon la revendication 1 ou 2, caractérisé en ce qu'au moins un des groupes R est un radical acyclique consistant en un groupe alkyle, alkényle ou alkynyle à chaine linéaire ou ramifiée, soit non substitué soit substitué par un ou plusieurs des groupes thio, halo, oxo, hydroxy, carbonyle,

carboxy, alkylcarbonyloxy, mercapto, sulfinyle, sulfonyle, imino, amino, cyano, nitro, hydroxyamino, nitroso, cycloalkyle, aryle, aralkyle, aryloxy, arylalkoxy, arylthio, aralkylthio, arylamino ou aralkylamino.

4. Procédé selon la revendication 1 ou 2, caractérisé en ce qu'au moins un des groupes R est un radical alicyclique consistant en un groupe aliphatique cyclique saturé ou insaturé, soit non-substitué soit substitué avec un ou plusieurs des substituants de la liste formée par les radicaux alkyle, alkényle, alkynyle, alkoxy, alkényloxy, alkynyloxy, alkylthio, alkénylthio, alkynylthio, halo, oxo hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino aralkylamino ou alkarylamino.

5. Procédé selon la revendication 1 ou 2, caractérisé en ce qu'au moins un des Groupes R est un radical aromatique consistant en un groupe phényle, naphthyle, biphényle, anthracyle ou phénantryle, soit non substitué soit substitué par un ou plusieurs des groupes alkyle, alkényle, alkynyle, alkoxy, alkényloxy, alkynyloxy, alkylthio, alkénylthio, alkynylthio, halo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, cycloalkyle, cycloalkaryle, alkaryle, alkaryloxy, alkarylthio ou alkarylamino.

6. Procédé selon la revendication 1 ou 2, caractérisé en ce qu'au moins un des groupes R est un radical hétérocyclique consistant en un groupe alicyclique ou aromatique contenant un ou plusieurs hétéroatomes consistant en oxygène, azote ou soufre dans le noyau, ce groupe pouvant être substitué par un ou plusieurs substituants consistant en les radicaux alkyle, alkényle, alkynyle, alkoxy, alkényloxy, alkynyloxy, alkylthio, alkénylthio, alkynylthio, halo, hydroxy, carbonyle, carboxy, alkylcarbonyloxy, alkylcarbonyle, carboxyalkyle, thio, mercapto, sulfinyle, sulfonyle, imino, amino, cyano, nitro, hydroxyamino, nitroso, aryle, aralkyle, alkaryle, aryloxy, arylalkoxy, alkaryloxy, arylthio, aralkylthio, alkarylthio, arylamino, aralkylamino et alkarylamino.

7. Procédé selon la revendication 1, caractérisé en ce qu'au moins un des groupes R est un groupe hétéroacyclique consistant en un radical acyclique à chaine linéaire ou ramifiée, saturé ou insaturé contenant un ou plusieurs hétéroatomes consistant en oxygène, azote ou soufre dans le noyau, ce groupe pouvant être substitué par un ou plusieurs substituants consistant en les radicaux halo, hydroxy, carbonyle, mercapto, sulfinyle, sulfonyle, imino, amino, cyano, nitro, hydroxyamino, nitroso, aryle, aryloxy, arylthio et arylamino.

8. Procédé selon la revendication 1 ou 2, caractérisé en ce qu'il comprend des motifs structuraux fondamentaux de formule:

$$M(O_3PO_xRCOOH)_2$$

dans laquelle M, x et R ont les mêmes significations que dans les revendications 1 et 2.

9. Procédé selon la revendication 1 ou 2, caractérisé en ce qu'il comprend des motifs structuraux fondamentaux de formule:

$$M(O_3PO_xRSO_3H)_2$$

dans laquelle M, x et R ont les mêmes significations que dans les revendications 1 et 2.

10. Procédé selon la revendication 1 ou 2, caractérisé en ce qu'il comprend des motifs structuraux fondamentaux de formule:

$$M(O_3PO_xRCN)_2$$

dans laquelle M, x et R ont les mêmes significations que dans les revendications 1 et 2.

11. Procédé selon la revendication 1 ou 2, caractérisé en ce qu'il comprend des motifs structuraux fondamentaux de formule:

$$M(O_3PO_xRSH)_2$$

dans laquelle M, x et R ont les mêmes significations que dans les revendications 1 et 2.

12. Procédé selon la revendication 6 ou 7, caractérisé en ce que l'hétéroatome est le soufre.

13. Procédé selon la revendication 6 ou 7, caractérisé en ce que l'hétéroatome est l'oxygène.

14. Procédé selon la revendication 6 ou 7, caractérisé en ce que l'hétéroatome est l'azote.

15. Procédé selon la revendication 3, caractérisé en ce qu'il comprend des groupes organiques de pontage entre couches contenant des motifs structuraux de formule:

$$M(O_3PO_xRO_xPO_3)$$

et dans laquelle M, x et R ont les mêmes significations que dans les revendications 1 et 2.

16. Procédé selon la revendication 1, 2, 8, 9, 10 ou 11 caractérisé en ce qu'il contient en outre 0,01 à 10% en poids d'un métal du groupe VIII.

17. Procédé selon la revendication 16, dans lequel le métal du groupe VIII est le palladium.

18. Application à la catalyse d'un polymère préparé selon l'une quelconque des revendications précédentes.

19. Application à la sorbtion de composés organiques d'un polymère préparé selon l'une quelconque des revendications 1 à 17.

20. Application à l'échange ionique d'un polymère préparé selon l'une quelconque des revendications 1 à 17.

21. Application à la complexation ionique d'un polymère préparé selon l'une quelconque des revendications 1 à 17.

22. Application à la formulation de compsoitions à libération contrôlée d'un polymère préparé selon l'une quelconque des revendications 1 à 17.

23. Application en tant qu'additifs de compositions polymères d'un polymère préparé selon l'une quelconque des revendications 1 à 17.

**Patentansprüche für die Vertragsstaaten: BE CH DE FR GB IT NL SE**

1. Ein festes anorganisches Polymeres mit Basisstruktur-Einheiten der Formel:

$$M(O_3PO_xR)_n$$

in welchem M eines oder mehrere der tetravalenten Elemente Wolfram, Uran, Titan, Thorium, Tellur, Zinn, Silicium, Rubidium, Plutonium, Praseodym, Blei, Osium, Niob, Molybdän, Mangan, Iridium, Hafnium, Germanium und Cer ist; R eine oder mehrere organische azyklische, alizyklische, heteroazyklische, heterozyklische oder aromatische Gruppen ist; x 0 oder 1 ist; und n 1 oder 2 ist, vorausgesetzt, daß dann, wenn n 1 ist, R mit einer unsubstituierten Phosphat- oder Phosphonat-Gruppe terminiert und mindestens 2 Kohlenstoffatome, welche die Phosphoratome trennen, enthält.

2. Ein festes anorganisches Polymer mit Basisstruktur-Einheiten der Formel:

$$M(O_3PO_xR)_n$$

in welcher M eines oder mehrere der tetravalenten Metalle Titan, Molybdän, Zinn, Cer, Hafnium, Blei, Thorium und Uran ist; R eine oder mehrere organische azyklische, alizyklische, heteroazyklische, heterozyklische oder aromatische Gruppen ist; x 0 oder 1 ist; und n 1 oder 2 ist, vorausgesetzt, daß, wenn n 1 ist, R mit einer unsubstituierten Phosphat- oder Phosphonat-Gruppe terminiert und mindestens 2 Kohlenstoffatome enthält, die die Phosphoratome trennen.

3. Ein Polymer nach Anspruch 1 oder 2, in welchem mindestens ein R eine azyklische Gruppe, bestehend aus einer geradkettigen oder verzweigten Alkyl-, Alkenyl- oder Alkinyl-Gruppe, ist, entweder unsubstituiert oder substituiert mit einer oder mehreren von Thio, Halo, Oxo, Hydroxy, Carbonyl, Carboxy, Alkylencarbonyloxy, Mercapto, Sulfinyl, Sulfonyl, Imino, Amino, Cyano, Nitro, Hydroxyamino, Nitroso, Cycloylkyl, Aryl Aralkyl, Aryloxy, Arylalkoxy, Arylthio, Aralkylthio, Arylamino, oder Aralkylamino.

4. Ein Polymer, wie in Anspruch 1 oder 2 beansprucht, in welchem mindestens ein R eine alizyklische Gruppe bestehend aus einer gesättigten oder ungesättigten zyklischen aliphatischen Gruppe ist, entweder substituiert oder unsubstituiert mit einer oder mehreren von Alkyl, Alkenyl, Alkinyl, Alkoxy, Alkenyloxy, Alkinyloxy, Alkylthio, Alkenylthio, Alkinylthio, Halo, Oxo, Hydroxy, Carbonyl, Carboxy, Alkylcarbonyloxy, Alkylcarboxyl, Carboxyalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino oder Alkarylamino.

5. Ein Polymer, wie in Anspruch 1 oder 2 beansprucht, in welchem mindestens ein R eine aromatische Gruppe bestehend aus Phenyl, Naphthyl, Biphenyl, Anthracyl oder Phenanthryl-Gruppe ist, entweder unsubstituiert oder substituiert mit einer oder mehreren von Alkyl, Alkenyl, Alkinyl, Alkoxy, Alkenyloxy, Alkinyloxy, Alkylthio, Alkenylthio, Alkinylthio, Halo, Hydroxy, Carbonyl, Carboxy, Alkylcarbonyloxy, Alkylcarbonyl, Carboxyalkyl, Thio, Mercapto, Sulfinyl, Sulfonyl, Imino, Amino, Cyano, Nitro, Hydroxyamino, Nitroso, Cycloalkyl, Cycloalkaryl, Alkaryl, Alkaryloxy, Alkarylthio, oder Alkarylamino.

6. Ein Polymer, wie in Anspruch 1 oder 2 beansprucht, in welchem mindestens ein R eine heterozyklische Gruppe bestehend aus einer alizyklischen oder aromatischen Gruppe ist, welche ein oder mehrere der Heteratome Sauerstoff, Stickstoff oder Schwefel im Ring enthält, wobei die Gruppe mit ein oder mehreren von Alkyl, Alkenyl, Alkinyl, Alkoxy, Alkenyloxy, Alkinyloxy, Alkylthio, Alkenylthio, Alkinylthio, Halo, Hydroxy, Carbonyl, Carboxy, Alkylcarbonyloxy, Alkylcarbonyl, Carbonylalkyl, Thio, Mercapto, Sulfinyl, Sulfonyl, Imino, Amino, Cyano, Nitro, Hydroxyamino, Nitroso, Aryl, Aryloxy, Arylthio, Aryloxy, Arylalkoxy, Alkaryloxy, Arylthio, Aralkylthio, Alkarylthio, Arylamino, Aralkylamino, und Alkarylamino substituiert sein kann.

7. Ein Polymer, wie in Anspruch 1 oder 2 beansprucht, in welchem mindestens ein R eine heterozyklische Gruppe bestehend aus einer verzweigten oder geradkettigen, gesättigten oder ungesättigten azyklischen Gruppe, welche ein oder mehrere der Heteroatome Sauerstoff, Stickstoff oder Schwefel in der Kette aufweist, wobei diese Gruppe mit einem oder mehreren von Halo, Hydroxy, Carbonyl, Mercapto, Sulfinyl, Sulfonyl, Imino, Amino, Cyano, Nitro, Hydroxyamino, Nitroso, Aryl, Aryloxy, Arylthio, und Arylamino substituiert sein kann.

8. Polymer, wie in Anspruch 1 oder 2 beansprucht, welches Basisstruktur-Einheiten der Formel besitzt:

$$M(O_3PO_xRCOOH)_2$$

und in welcher M, x und R die gleiche Bedeutung wie in den Ansprüchen 1 und 2 besitzen.

9. Ein Polymer, wie in Anspruch 1 oder 2 beansprucht, welches Basisstruktur-Einheiten der Formel besitzt:

$$M(O_3PO_xRSO_3H)_2$$

und in welcher M, x und R die gleiche Bedeutung wie in den Ansprüchen 1 und 2 besitzen.

10. Ein Polymer, wie in Anspruch 1 oder 2 beansprucht, welches Basisstruktur-Einheiten der Formel besitzt:

$$M(O_3PO_xRCN)_2$$

in welcher M, x und R die gleiche Bedeutung wie in den Ansprüchen 1 und 2 besitzen.

11. Ein Polymer, wie in Anspruch 1 oder 2 beansprucht, welches Basisstruktur-Einheiten der Formel besitzt:

$$M(O_3PO_xRSH)_2$$

in welcher M, x und R die gleiche Bedeutung wie in den Ansprüchen 1 und 2 besitzen.

12. Ein Polymer, wie in den Ansprüchen 6 oder 7 beansprucht, in welchem ein Heteroatom Schwefel ist.

13. Ein Polymer, wie in Anspruch 6 oder 7 beansprucht, in welchem ein Heteroatom Sauerstoff ist.

14. Ein Polymer, wie in Anspruch 6 oder 7 beansprucht, in welchem ein Heteroatom Stickstoff ist.

15. Ein Polymer, wie in Anspruch 3 beansprucht, welches schichtüberbrückende organische Gruppen bietet, welche Struktureinheiten der Formel enthalten:

$$M(O_3PO_xRO_xPO_3)$$

und in welcher M, x und R die gleiche Bedeutung wie in Anspruch 3 haben.

16. Ein Polymer, wie in Anspruch 1, 2, 8, 9, 10 oder 11 beansprucht, welches weiterhin zwischen 0.01 bis etwa 10 Gew.-% eines Metalls der Gruppe VIII aufweist.

17. Ein Polymer, wie in Anspruch 16 beansprucht, in welchem das Metall der Gruppe VIII Palladium ist.

18. Die Verwendung eines Polymeren wie in irgendeinem der vorhergehenden Ansprüche beansprucht, zur Katalyse.

19. Die Verwendung eines Polymeren, wie in irgendeinem der Ansprüche 1 bis 17 beansprucht, zur Sorption von organischen Verbindungen.

20. Die Verwendung eines Polymeren, wie in irgendeinem der Ansprüche 1 bis 17 beansprucht, zum Ionenaustausch.

21. Die Verwendung eines Polymeren, wie in irgendeinem der Ansprüche 1 bis 17 beansprucht, zum Ionen-Komplexieren.

22. Die Verwendung eines Polymeren, wie in irgendeinem der Ansprüche 1 bis 17 beansprucht, zur Formulierung von Verbindungen mit gesteuerter Abgabe.

23. Die Verwendung eines Polymeren, wie in einem der Ansprüche 1 bis 17 beansprucht, als Additiv zu Polymer-Zusammensetzungen.

**Patentansprüche für den Vertragsstaat: AT**

1. Ein Verfahren zur Herstellung eines neuen anorganischen Polymers, dadurch gekennzeichnet, daß das Polymer Basisstruktur-Einheiten der Formel

$$M(O_3PO_xR)_n$$

hat und daß für M eines oder mehrere der tetravalenten Elemente Wolfraum, Uran, Titan, Thorium, Tellur, Zinn, Silicium, Rubidium, Plutonium, Praseodym, Blei, Osmium, Niob, Molybdän, Mangan, Iridium, Hafnium, Germanium und Cer verwendet wird, für R eine oder mehrere organische azyklische, alizyklische, heteroazyklische, heterozyklische oder aromatische Gruppen verwendet werden, x 0 der 1 gewählt wird

und n 1 oder 2 gewählt wird, vorausgesetzt, daß dann, wenn n 1 ist, R mit einer unsubstituierten Phosphat- oder Phosphonat-Gruppe terminiert und mindestens zwei Kohlenstoffatome, welche die Phosphoratome trennen, enthält.

2. Ein Verfahren zur Herstellung eines neuen anorganischen Polymers, dadurch gekennzeichnet, daß das Polymer Basisstruktur-Einheiten der Formel

$$M(O_3PO_xR)_n$$

hat und daß für M eines oder mehrere der tetravalenten Metalle Titan, Molybdän, Zinn, Cer, Hafnium, Blei, Thorium und Uran verwendet werden, für R eine oder mehrere organische azyklische, alizyklische, heteroazyklische, heterozyklische oder aromatische Gruppen verwendet werden, x 0 oder 1 gewählt wird und n 1 oder 2 gewählt wird, vorausgesetzt, daß, wenn n 1 ist, R mit einer unsubstituierten Phosphat- oder Phosphonat-Gruppe terminiert und mindestens zwei Kohlenstoffatome enthält, die die Phosphoratome trennen.

3. Ein Verfahren nach Anspruch 1 oder 2, in welchem mindestens für ein R eine azyklische Gruppe, bestehend aus einer geradkettigen oder verzweigten Alkyl-, Alkenyl- oder Alkinyl-Gruppe, entweder unsubstituiert oder substituiert mit einer oder mehreren von Thio, Halo, Oxo, Hydroxy, Carbonyl, Carboxy, Alkylcarbonyloxy, Mercapto, Sulfinyl, Sulfonyl, Imino, Amino, Cyano, Nitro, Hydroxyamino, Nitroso, Cycloalkyl, Aryl, Aralkyl, Aryloxy, Arylalkoxy, Arylthio, Aralkylthio, Arylamino oder Aralkylamino verwendet wird.

4. Ein Verfahren nach Anspruch 1 oder 2, in welchem mindestens für ein R eine alizyklische Gruppe, bestehend aus einer gesättigten oder ungesättigten zyklischen aliphatischen Gruppe, entweder substituiert oder unsubstituiert mit einer oder mehreren von Alkyl, Alkenyl, Alkinyl, Alkoxy, Alkenyloxy, Alkinyloxy, Alkylthio, Alkenylthio, Alkinylthio, Halo, Oxo, Hydroxy, Carbonyl, Carboxy, Alkylcarbonyloxy, Alkylcarboxyl, Carboxylalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino oder Alkarylamino verwendet wird.

5. Eine Verfahren nach Anspruch 1 oder 2, in welchem mindestens für ein R eine aromatische Gruppe, bestehend aus Phenyl, Naphthyl, Biphenyl, Anthracyl oder Phenanthryl-Gruppe, entweder unsubstituiert oder substituiert mit einer oder mehreren von Alkyl, Alkenyl, Alkinyl, Alkoxy, Alkenyloxy, Alkinyloxy, Alkylthio, Alkenylthio, Alkinylthio, Halo, Hydroxy, Carbonyl, Carboxy, Alkylcarbonyloxy, Alkylcarbonyl, Carboxyalkyl, Thio, Mercapto, Sulfinyl, Sulfonyl, Imino, Amino, Cyano, Nitro, Hydroxyamino, Nitroso, Cycloalkyl, Cycloalkaryl, Alkaryl, Alkaryloxy, Alkarylthio oder Alkarylamino verwendet wird.

6. Ein Verfahren nach Anspruch 1 oder 2, in welchem mindestens für ein R eine heterozyklische Gruppe, bestehend aus einer alizyklischen oder aromatischen Gruppe, verwendet wird, welche ein oder mehrere der Heteroatome Sauerstoff, Stickstoff oder Schwefel im Ring enthält, wobei die Gruppe mit ein oder mehreren von Alkyl, Alkenyl, Akinyl, Alkoxy, Alkenyloxy, Alkinyloxy, Alkylthio, Alkenylthio, Alkinylthio, Halo, Hydroxy, Carbonyl, Carboxy, Alkylcarbonyloxy, Alkylcarbonyl, Carbonylalkyl, Thio, Mercapto, Sulfinyl, Sulfonyl, Imino, Amino, Cyano, Nitro, Hydroxyamino, Nitroso, Aryl, Aralkyl, Alkaryl, Aryloxy, Arylalkoxy, Alkaryloxy, Arylthio, Aralkylthio, Alkarylthio, Arylamino, Aralkylamino und Alkarylamino substituiert sein kann.

7. Ein Verfahren nach Anspruch 1 oder 2, in welchem mindestens für ein R eine heteroazyklische Gruppe bestehend aus einer verzweigten oder geradkettigen, gesättigten oder ungesättigten azyklischen Gruppe verwendet wird, welche ein oder mehrere der Heteroatome Sauerstoff, Stickstoff oder Schwefel in der Kette aufweist, wobei diese Gruppe mit einem oder mehreren von Halo, Hydroxy, Carbonyl, Mercapto, Sulfinyl, Sulfonyl, Imino, Amino, Cyano, Nitro, Hydroxyamino, Nitroso, Aryl, Aryloxy, Arylthio und Arylamino substituiert sein kann.

8. Eine Verfahren nach Anspruch 1 oder 2, dadurch gekennzeichnet, daß das Polymer Basisstruktur-Einheiten der Formel besitzt.

$$M(O_3PO_xRCOOH)_2$$

in welcher M, x und R die gleiche Bedeutung wie in den Ansprüchen 1 und 2 besitzen.

9. Eine Verfahren nach Anspruch 1 oder 2, dadurch gekennzeichnet, daß das Polymer Basisstruktur-Einheiten der Formel besitzt

$$M(O_3PO_xRSO_3H)_2$$

und in welcher M, x und R die gleiche Bedeutung wie in den Ansprüchen 1 und 2 besitzen.

10. Ein Verfahren nach Anspruch 1 oder 2, dadurch gekennzeichnet, daß das Polymer Basisstruktur-Einheiten der Formel besitzt

$$M(O_3PO_xRCN)_2$$

in welcher M, x und R die gleiche Bedeutung wie in den Ansprüchen 1 und 2 besitzen.

11. Ein Verfahren nach Anspruch 1 oder 2, dadurch gekennzeichnet, daß das Polymer Basisstruktur-Einheiten der Formel besitzt

$$M(O_3PO_xRSH)_2$$

in welcher M, x und R die gleiche Bedeutung wie in den Ansprüchen 1 und 2 besitzen.

12. Ein Verfahren nach den Ansprüchen 6 oder 7, in welchem Schwefel als ein Heteroatom verwendet wird.

13. Ein Verfahren nach Anspruch 6 oder 7, in welchem Sauerstoff als ein Heteroatom verwendet wird.

14. Ein Verfahren nach Anspruch 6 oder 7, in welchem Stickstoff als ein Heteroatom verwendet wird.

15. Ein Verfahren nach Anspruch 3, dadurch gekennzeichnet, daß schichtüberbrückende organische Gruppen, welche Struktureinheiten der Formel enthalten

$$M(O_3PO_xRO_xPO_3)$$

und in welcher M, x und R die gleiche Bedeutung wie in Anspruch 3 haben, im Polymer vorgesehen werden.

16. Ein Verfahren nach Anspruch 1, 2, 8, 9, 10 oder 11, dadurch gekennzeichnet, daß zwischen etwa 0,01 bis etwa 10 Gew.% eines Metalls der Gruppe VIII im Polymer vorgesehen werden.

17. Ein Verfahren nach Anspruch 16, in welchem Palladium als Metall der Gruppe VIII verwendet wird.

18. Die Verwendung eines Polymeren, das nach einem Verfahren gemäß einem der vorhergehenden Ansprüche hergestellt worden ist, zur Katalyse.

19. Die Verwendung eines Polymeren, das nach einem Verfahren gemäß einem der Ansprüche 1 bis 17 hergestellt worden ist, zur Sorption von organischen Verbindungen.

20. Die Verwendung eines Polymeren, das nach einem Verfahren gemäß einem der Ansprüche 1 bis 17 hergestellt worden ist, zum Ionenaustausch.

21. Die Verwendung eines Polymeren, das nach einem Verfahren gemäß einem der Ansprüche 1 bis 17 hergestellt worden ist, zum Ionen-Komplexieren.

22. Die Verwendung eines Polymeren, das nach einem Verfahren nach einem der Ansprüche 1 bis 17 hergestellt worden ist, zur Formulierung von Verbindungen mit gesteuerter Abgabe.

23. Die Verwendung eines Polymeren, das nach einem Verfahren nach einem der Ansprüche 1 bis 17 hergestellt worden ist, als Additiv zu Polymer-Zusammensetzungen.

Fig.1

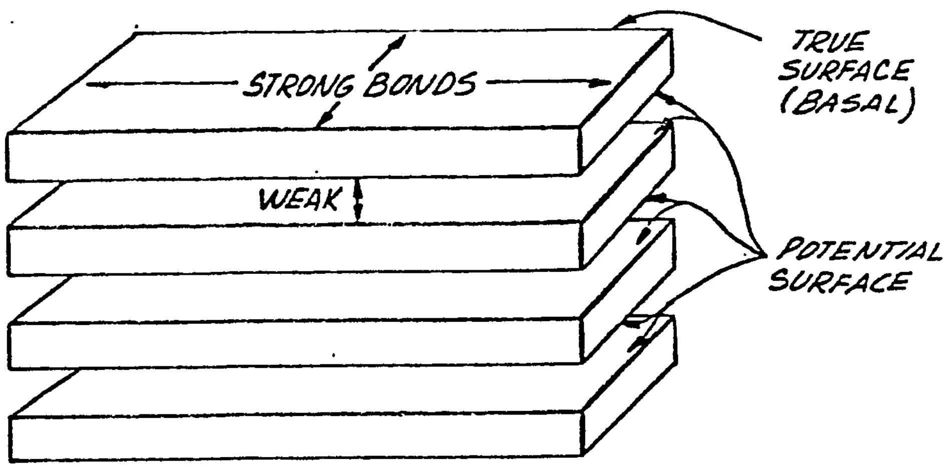
STRONG BONDS
WEAK
TRUE SURFACE (BASAL)
POTENTIAL SURFACE

Fig.2

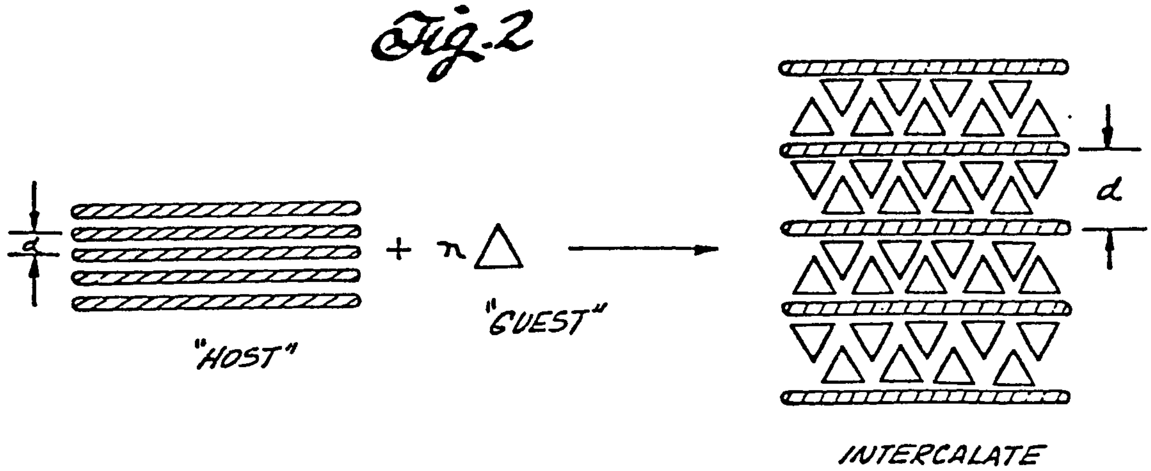
d
+ n
"HOST"
"GUEST"
d
INTERCALATE

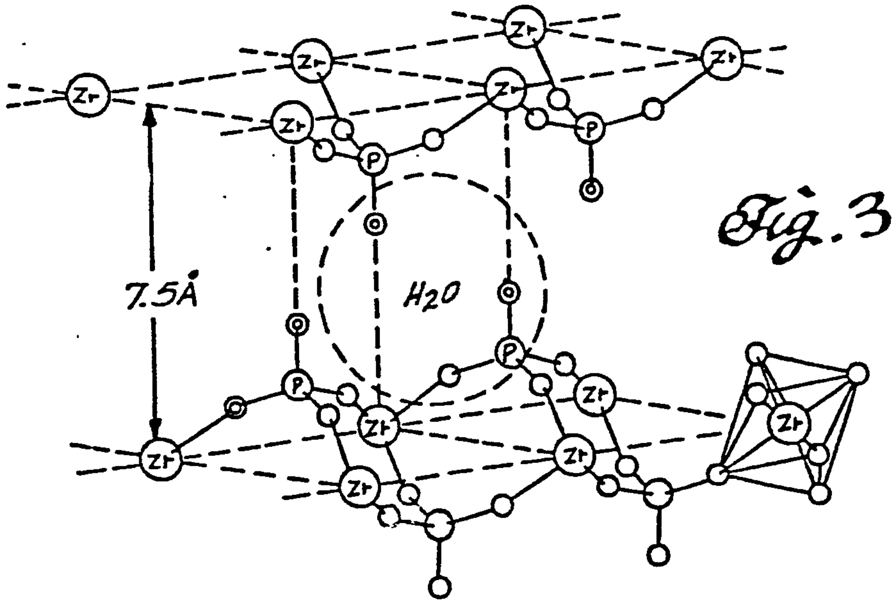


Fig. 3

Fig. 5

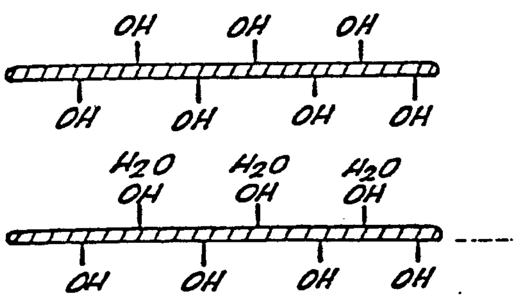


Fig. 4

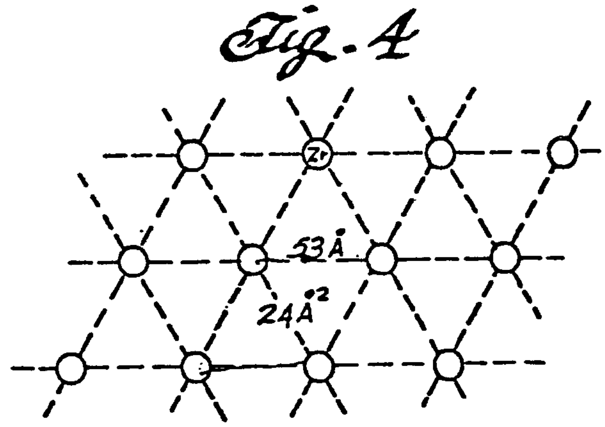


Fig. 6

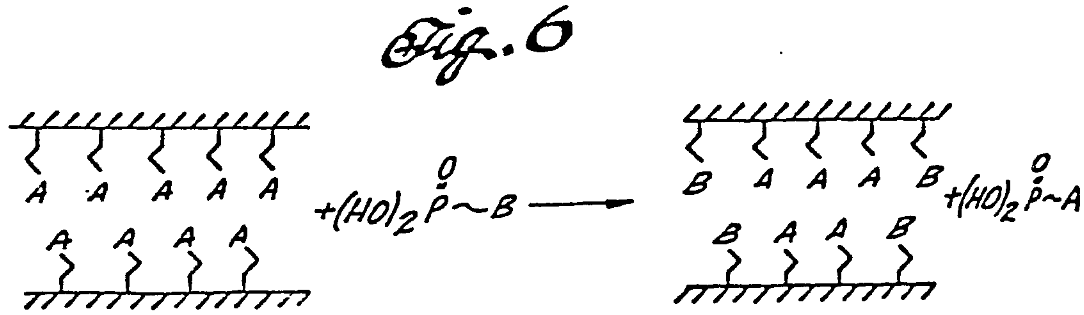

Fig. 7

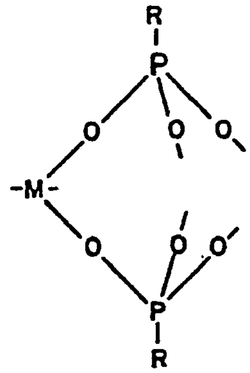

Fig. 8